(19) Europäisches Patentamt
European Patent Office
Office européen des brevets

(11) EP 1 569 953 B1

(12) EUROPEAN PATENT SPECIFICATION

(45) Date of publication and mention of the grant of the patent:
**29.09.2010 Bulletin 2010/39**

(21) Application number: **03773592.5**

(22) Date of filing: **25.11.2003**

(51) Int Cl.:
***C07K 5/06*** (2006.01) ***A61K 38/06*** (2006.01)
***A61P 9/06*** (2006.01)

(86) International application number:
**PCT/DK2003/000805**

(87) International publication number:
**WO 2004/048400 (10.06.2004 Gazette 2004/24)**

# (54) PEPTIDE GAP JUNCTION MODULATORS

PEPTIDSPALTVERBINDUNGS-MODULATOREN

MODULATEURS DE LA JONCTION COMMUNICANTE DE PEPTIDES

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **25.11.2002 US 428973 P**

(43) Date of publication of application:
**07.09.2005 Bulletin 2005/36**

(73) Proprietor: **Zealand Pharma A/S**
**2600 Glostrup (DK)**

(72) Inventors:
- **LARSEN, Bjarne, Due**
**DK-2720 Vanlose (DK)**
- **KNUDSEN, Carsten, Boye**
**DK-2670 Greve (DK)**
- **PETERSEN, Jorgen, Soberg**
**DK-3150 Hellebaek (DK)**

(74) Representative: **Kiddle, Simon John et al**
**Mewburn Ellis LLP**
**33 Gutter Lane**
**London**
**EC2V 8AS (GB)**

(56) References cited:
**WO-A-02/077017**

- **REICHARDT PETER ET AL: "Identification and Quantification of in vitro Adduct Formation Between Protein Reactive Xenobiotics and a Lysine-Containing Model Peptide" ENVIRONMENTAL TOXICOLOGY, vol. 18, no. 1, 2003, pages 29-36, XP002275870 -& DATABASE HCAPLUS [Online] REICHARDT, PETER ET AL: "Identification and quantification of in vitro adduct formation between protein reactive xenobiotics and a lysine-containing model peptide" retrieved from STN Database accession no. 2003:116032 HCAPLUS XP002275996**
- **TAKASHI SEKI ET AL: "Delta-Acetyl-L-ornithyl-Beta-alanine Methyl Ester Hydrochloride, an Intermolecule Type Sweetener" AGRIC. BIOL. CHEM., vol. 54, no. 7, 1990, pages 1811-1818, XP002275871 -& DATABASE HCAPLUS [Online] SEKI, TAKASHI ET AL: "Studies on flavored peptides. Part IX. <SYM100>-Acetyl-L-ornithyl-<SYM98>-alanine methyl ester hydrochloride, an intermolecule type sweetener" retrieved from STN Database accession no. 1990:570657 XP002275997**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 569 953 B1

Printed by Jouve, 75001 PARIS (FR)

## Description

### FIELD OF THE INVENTION

The invention relates to orally available dipeptides capable of modulating intracellular gap junctional communication. The invention also relates to methods of using the peptides to modulate such communication, to the use of the peptides for the manufacture ofmedicaments for the prevention and/or treatment of conditions associated with said communication and to pharmaceutical compositions comprising said dipeptides.

### BACKGROUND

There is increasing recognition that intercellular communication is essential for cellular homeostasis, proliferation and differentiation. Such communication is believed to be facilitated by gap junctions. These structures are thought to be a route for coupling cells and permiting "cross-talk". See generally, Sperelakis, N., (1989) Cell Interactions and Gap Junctions by N. Sperelakis, William C. Cole (Editor).

There have been efforts to understand the structure and function of gap junctions. For instance, there have been reports that such junctions are a type of complex formed between adjacent cells. Most gap junctions are thought to consist of aggregated channels that directly link the interiors (cytoplasms) of neighboring cells. In adult mammals, gap junctions are found in most cell types with the exception of circulating blood elements.

More specifically, there is acknowledgement that gap junctions are specialized regions of the cell membrane with clusters of hundreds to thousands of densely packed gap junction channels (comprising two hemichannels or connexins). Many are thought to directly connect the cytoplasmic compartments of two neighboring cells. The gap junction channel can switch between an open and a closed state. In the open state ions and small molecules are thought to pass through the pore. The conduction of electrical impulses and intercellular diffusion of signaling molecules take place through the gap junctions.

The "cross-talk" between gap junctions has been referred to as gap junctional intracellular communication (GJIC), which is believed to play an important role in the regulation of cell metabolism, proliferation, cell-to-cell signaling, and tissue integrity.

For instance, GJIC is thought to permit rapid equilibration of nutrients, ions, and fluids between cells. Gap junctions are also thought to serve as electrical synapses in electrically excitable cells. In many tissues, electrical coupling is thought to permit more rapid cell-to-cell transmission of action potentials than chemical synapses. In cardiomyocytes and smooth muscle cells, for instance, this is thought to assist synchronous contraction.

There have been reports of other functions mediated by GJIC. For example, GJIC is thought to enhance the responsiveness of tissues to external stimuli. Second messengers are generally believed to be small enough to pass from hormonally activated cells to quiescent cells through junctional channels and activate the latter.

Additionally, there have been reports that gap junctions may provide intercellular pathways for chemical and/or electrical developmental signals and assist in defining the boundaries of developmental compartments. It has been disclosed that GJIC occurs in specific patterns in embryonic cells and the impairment of GJIC has been related to developmental anomalies and the teratogenic effects of many chemicals. Further, GJIC is thought to assist coordination of cell activities.

Some reports have established a link between abnormalities in GJIC and a range of disease states has been established both *in vitro* and *in vivo.* For example, there is thought to be a link between abnormalities in connexins and heart disease. Several studies of the expression and distribution of Cx43 in hearts describe a reduced degree of Cx43 expression and a changed pattern of distribution for this gap junction protein. See Kaprielian, R. R., et al. (1998) Circulation 97: 651-660; and Saffitz, J. E., et al., (1999) Cardiovasc Res. 42: 309-317.

Accordingly, there is recognition in the field of a relationship between a malfunction or absence of gap junctions and an increased risk of arrhythmias. There is thought to be a further relationship between altered connexin expression/distribution and chronic heart disease.

Attempts to analyze peptides that influence GJIC have been made. For instance, a group of peptides (the antiarrhythmic peptides) have been disclosed with capacity to increase gap junction conductance in the heart. In particular, a hexapeptide with a molecular weight of 470D was reported to have been isolated from bovine atria. In neonatal rat cardiomyocytes, it was reported that the peptide could convert fibrillation induced by ouabain, calcium, or potassium, to normal rhythm. In addition, the peptide has been reported to convert arrhythmic movement of isolated rat atria induced by the combination of potassium and acetylcholine to normal rhythm. This peptide is often referred to as antiarrhytmic peptide (AAP). See eg., Aonuma, S., et al. (1980) Chem Pharm Bull (Tokyo) 28: 3332-3339

There is increasing understanding that AAP is an important peptide with capacity to modulate GJIC in the heart.

For example, in cell culture, AAP has been shown to increase the number of beating centers, the relative content of spreading cells, and protein synthesis. See Aonuma, S., et al. (1980) Chem Pharm Bull (Tokyo) 28: 3340-3346.

In other studies, the antiarrhythmic effect of AAP observed in *vitro* has been confirmed. It has been reported that AAP is effective against $CaCl_2$-, oubain- and acotinine-induced arrhythmia in mice. See Ronsberg, M.A., et al. (1986) Med. Sci. 14: 350-351.

The AAP sequence has been disclosed. See eg., Aonuma, S., et al. (1982) J Pharmacobiodyn. 5: 40-48.

There have been efforts to prepare a variety of AAP derivatives. See eg., Dikshit, M., et al. (1988) Indian J. Exp. Biol. 26: 874-876; Kohama, Y., et al. (1987) Chem. Pharm. Bull. (Tokyo) 35: 3928-3930; and Kohama, Y., et al. (1988) Chem. Pharm. Bull. (Tokyo) 36: 4597-4599).

For instance, one such derivative, AAP10, is believed to impact GJIC. See Dhein, S., et al. (1994) Naunyn Schmiedebergs Arch Pharmacol. 350: 174-184; and Muller, A., et al. (1997) Eur. J. Pharmacol. 327: 65-72. It has been reported that AAP improves GJIC, reduces action potential dispersion and improves conductance.

There is increasing understanding that many of the antiarrhythmic peptides positively impact GJIC, often without affecting action potential duration or shape. Further, many of such peptides are thought to lack undesirable proarrhythmic side-effects. Such effects are thought to limit the use of many currently available antiarrhythmic drugs. Moreover, AAP, as well as certain AAP derivatives, are thought to have some undesired features, e.g., low stability and a need for high doses before therapeutic efficacy is achieved.

Many potential important peptides suffer from a lack of acceptable oral availability. That is, the peptides are degraded in the gastrointestinal tract, enterocytes, or both. In such cases, the peptides are often administered to subjects by more painful and less convenient intravenous routes.

There have been attempts to improve the oral availability of certain compounds by enhancing contact with an intestinal peptide transporter protein called PepT1. Much is known about the PepT1 transporter system. See Bailey, P.D., et al. (2000) Angew. Chem. Int. Ed. 39:506; and references cited therein.

It would be desirable to have more effective peptide modulators of GJIC. It would be especially desirable to have peptide modulators that are orally available.

Seki et al (Agric-Biol. Chem., 54(7):1811-1818, 1990) discloses small peptide derivatives for use as sweetners.

## SUMMARY OF THE INVENTION

The invention generally relates to peptides that modulate gap junction intercellular communication (GJIC) as set out in the claims. Preferred peptides are orally available. The invention has a wide spectrum of useful applications including use in the treatment or prevention of pathologies associated with impaired GJIC.

Accordingly, in a first aspect, the present invention provides a peptide for use in therapy represented by the general formula I:

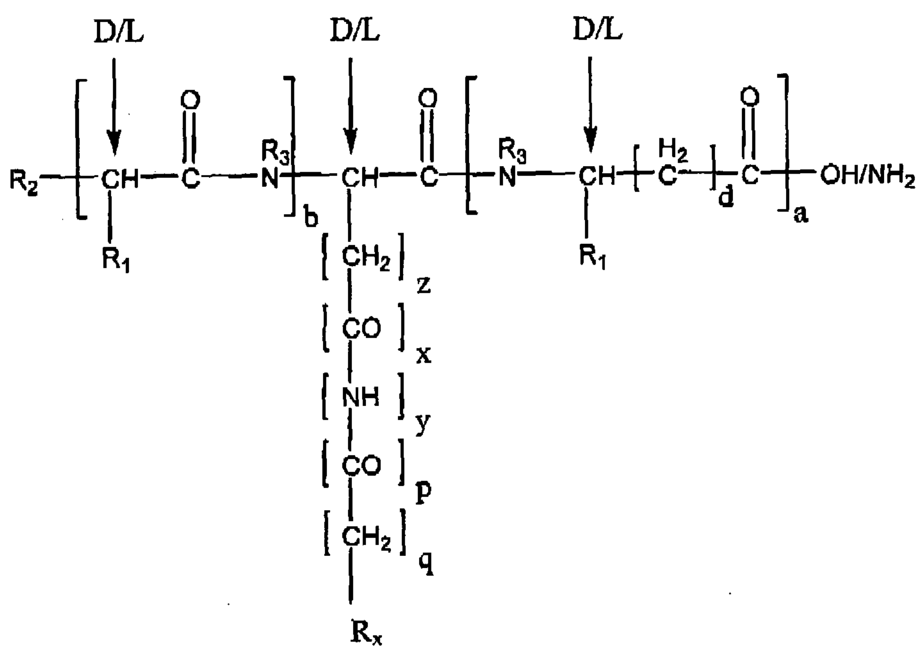

wherein:

a is 1 and b is 0; or
b is 1 and a is 0;
d is 0-8; and
z is 1-7; and
x is 1, y and q are 1, and p is 0; or
p is i , x and q are 0, and y is 1;
and further wherein,
if $R_1$ is H then d is 0-8; or
if $R_1$ is not H then d is 0;
wherein $R_1$ is the side chain of an amino acid selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine;
wherein $R_2$ is selected from the group consisting of $NH_2$, NHR, $NR_2$, $NR_3{}^{+}H$, OH, SH, RO, RS, RSO, $RSO_2$, $CO_R$, CSR, COOH, COOR, $CONH_2$, CONHR, CONR2, OCOR, and SCOR, wherein R = alkyl, alkenyl, aryl, aralkyl, or cycloalkyl;
wherein $R_3$ is H or $CH_{3;}$ and
wherein $R_X$ is a hydrophobic group;
or a pharmaceutically acceptable salt thereof.

Further, the invention concerns a peptide represented by the general formula II:

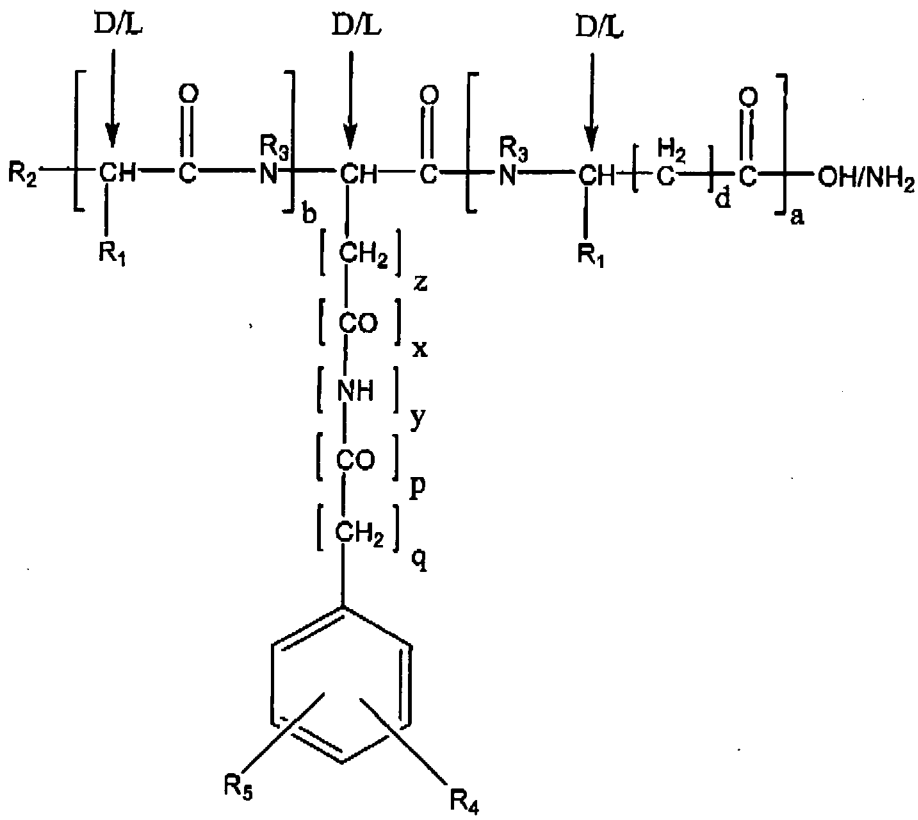

wherein:

a is 1 and b is 0; or

b is 1 and a is 0;

d is 0-8; and

z is 1-7; and

x is 1, y and q are 1, and p is 0; or

p is 1, x and q are 0, and y is 1;
and further wherein,
if $R_1$ is H then d is 0-8; or
if $R_1$ is not H then d is 0;
wherein $R_1$ is the side chain of an amino acid selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine;
wherein $R_2$ is selected from the group consisting of $NH_2$, NHR, $NR_2$, $NR_3{}^+H$, OH, SH, RO, RS, RSO, $RSO_2$, $CO_R$, CSR, COOH, COOR, $CONH_2$, CONHR, $CONR_2$, OCOR, and SCOR, wherein R = alkyl, alkenyl, aryl, aralkyl, or cycloalkyl;
wherein $R_3$ is H or $CH_{3:}$
wherein $R_4$ and $R_5$ are independently selected from the group consisting of H, alkyl, alkenyl, aryl, aralkyl, halogen, CN, $NO_2$, alkoxy, aryloxy, aralkyloxy, thioalkoxy, thioaryloxy, thioaralkyloxy, $+S(CH_3)_2$, $SO_3H$, $SO_2R$, $NH_2$, NHR, $NR_2$, $+NR_3$, OH, SH, COOH, COOR, $CONH_2$, CONHR, $CONR_2$, $CH_2OH$, NCO, NCOR, NHOH, $NHNH_2$, NHNRH, $CH_2OCOR$, $CH_2OCSR$, COR, CSR, CSOR, $CF_3$, and $CCl_3$, and wherein R is alkyl, alkenyl, aryl, aralkyl, or cycloalkyl;
or a pharmaceutically acceptable salt thereof.

Such peptides may also comprise a peptide bond that is alkylated or otherwise modified to stabilize the peptide against enzymatic degradation and/or may comprise D-amino acids.

More preferred peptides as represented by Formula I and II are orally available to a mammal, and particularly, to a human subject. Various assays to establish oral availability are known in the field and include, but are not limited to, the following specific tests.

Oral availability assays

One assay ("the Bailey assay") is a predictive assay and is generally followed in accordance with the invention by other empirical assays, described further below. In the Bailey assay, candidate peptides are virtually aligned with a substrate template that is modeled to represent a compound, which binds to PepT1, to identify peptides likely to bind to PepT1 *in vitro* and *in vivo.* The Bailey assay can be used to facilitate detection of orally available peptides of the invention.

In one embodiment, the peptides according to Formula I and II are used in substantial agreement with this substrate template model (e.g., the peptides show substantially the same three-dimensional conformation as previously identified substrates which bind to the hPepT1 with high affinity) and therefore are good candidates for peptides, which are orally available. Thus, such peptides are typically dipeptides.

More preferred peptides are those which exhibit at least the following four properties ascribed to high affinity substrates for PepT1; i.e., from the N- to the C-terminus of the peptide, the peptide comprises: 1) a strong binding site for an N-terminal $NH_3{}^+$ group; 2) a hydrogen bond to the carbonyl group of the first peptide bond; 3) a hydrophobic pocket preferably featuring strong directional vector and 4) a carboxylate binding site. Preferably, the first carboxylate binding site is followed by a second carboxylate binding site according to the model. See, e.g., Bailey, P.D., et al, (2000) *supra.*

One empirical assay to validate the predictive Bailey assay described above, is a "standard *in vivo* oral availability assay". In this assay, a peptide is orally administered to a mammal and blood samples are taken over time. The concentration of the peptide is determined at different time intervals using standard quantitation assays such as LC/MS/MS to calculate an area under the curve (AUC) from a plot of plasma protein concentration vs. time, using routine methods known in the art. Preferably, different doses of peptides are administered to a plurality of animals in parallel to identify those peptides that show a dose-proportional increase in maximum plasma concentration and AUC values. As a control, the same concentrations of peptide may be administered intravenously and the area under AUC obtained for oral administration can be compared to the AUC obtained for intravenous administration. See, e.g., Milo Gibal (1991) Biopharmaceutics and Pharmacology, 4th edition (Lea and Sediger). In a preferred embodiment peptides with good oral availability are those, in which the dose normalised AUC after oral administration represents at least 20% of the dose normalised AUC after intravenously administration.

Peptides which show good oral availability according to the standard *in vivo* oral availability assay described above, may or may not be in substantial agreement with the Bailey assay.

It will often be useful to perform a second empirical assay to further validate orally available peptides as represented by Formula I or II. In one such assay, the ability of the peptide to bind to the hPepT1 transporter or a

functionally active fragment thereof is determined. Preferably, peptides according to the invention have good affinity for an hPepT1 transporter or a biologically active fragment thereof. Preferably, the $K_i$ of such peptides is less than about 20 mM, more preferably, is less than about 10 mM, and still more preferably, is less than about 5 mM, or less than about 1 mM.

An hPepT1 binding assay may be performed as an initial screen for peptides which are then screened for in a standard *in vivo* oral availability assay and/or may be performed to confirm the results of a standard *in vivo* oral availability assay; however, the standard *in vivo* oral availability assay provides the most meaningful test of the peptide oral availability. Additionally preferred peptides as represented by Formula I above, exhibit a good half-life according to what is referred to herein as an "*in vitro* plasma stability assay" or related phrase. Such peptides may be in substantial agreement with the foregoing PepT1 substrate template model (Bailey assay). However, for some peptides there may be little or no agreement with the model. Peptides that show a good stability in the assay have in one embodiment a half-life of more than about 48 hours, such as more than 24 hours, for example more than 12 hours, such as more than 6 hours, for example more than 3 hours, such as more than 1 hour, for example more than 30 minutes. In this embodiment, the peptides of the invention show enhanced stability in the bloodstream.

Peptides within the scope of the present invention are in one embodiment represented herein with free N-terminal and/or C-terminal group. These groups may remain free for some invention uses. However, in another embodiment, the peptides can feature blocked C-terminal groups and free N-groups. Alternatively, such peptides may have blocked N-groups and free C-terminal groups, or blocked N- and C-terminal groups. The nature of the terminal groups at either side of the molecule is not critical so long as the peptides are able to bind with satisfactory affinity to hPepT1 such that the peptides and/or are orally available. As discussed, "satisfactory affinity" for the hPepT1 transporter can be predicted, in advance, by determining if a subject peptide is in substantial agreement with the substrate template model for binding to the hPepT1 carrier. See Bailey, P.D., et al. (2000), *supra.*

Additionally, amino acids residues within the dipeptides may be D- or L-amino acids. In certain aspects, to enhance stability of the compounds, D- amino acids are preferred.

The peptides according to the invention have a wide variety of important uses and advantages.

For instance, such peptides may be used for preventing and/or treating conditions associated with impaired gap junction function resulting in reduced intercellular communication or overcoupling of intercellular communication or misregulated cellular communication and be used for the manufacture of a medicament for preventing an/or treating conditions associated with impaired gap junction function. In one aspect, the invention provides a method of administering to a patient having, or at risk of developing such a condition, a therapeutically effective amount of any of the peptides described above. Preferably, administration is oral. In one preferred aspect, a patient is a human being.

Examples of conditions which can be treated include, but are not limited to, cardiovascular disease, inflammation of airway epithelium, disorders of alveolar tissue, bladder incontinence, impaired hearing due to diseases of the cochlea, endothelial lesions, diabetic retinopathy and diabetic neuropathy, ischemia of the central nervous system and spinal cord, dental tissue disorders including periodontal disease, kidney diseases, failures of bone marrow transplantation, wounds, erectile dysfunction, urinary bladder incontinence, neuropathic paln, subchronic and chronic inflammation, cancer and failures of bone marrow and stern cell transplantation, conditions which arise during transplantation of cells and tissues or during medical procedures such as surgery.

These treatments may employ pharmaceutical compositions comprising any of the peptides described above and a pharmaceutically acceptable carrier. Preferably, the carrier is sterile, pyrogen-free and virus-free. Still more preferably, the composition is orally adminstrable.

BRIEF DESCRIPTION OF THE DRAWINGS

Figures 1A and 1B show exemplary synthesis schemes for generating peptides according to certain aspects of the invention.

Figure 2 shows the effect of Compound 4, Compound 22, Compound 23 and Compound 95 on ALP (Alkaline Phosphatase) in human osteoblasts.

## DETAILED DESCRIPTION OF THE INVENTION

As discussed, the invention relates to orally available peptides that modulate gap junction intercellular communication (GJIC). The invention has a wide spectrum of useful applications including use in the treatment or prevention of pathologies associated with impaired GJIC. Particular invention peptides are represented by Formula I above.

More specific peptides according to the invention are represented by the following general formula II as defined

above and in the claims.

As discussed, the peptide can include a free N-terminal, or a free C-terminal, or both.

Preferably, the peptide comprises at least one hydrophobic group and at least one hydrogen bond group. In one aspect of the invention the three dimensional structure of the peptide the distance between the at least one hydrophobic group and the at least one hydrogen bonding group Is from about 4 Ångstrøms to about 12 Ångstrøms, preferably 5 to about 10 Ångstrøms. More preferably, the hydrophobic group is an aromatic group, including, but not limited to, a benzoyl or benzyl group, or substituted, or modified forms thereof. Substituted benzoyl or benzyl groups preferably comprise substituents at the 4- position, More preferably, the substitutents have a radius of 3-11 Ångstrøms In the sphere taken up by the substitutent. Suitable substituents Include, but are not limited to: methyl, ethyl, t-butyl, c-hexyl, phenyl, n-butyl, n-hexyl, n-octyl, ethoxy, t-butoxy, phenoxy, butoxy, benzyloxy, n-hexyloxy, n-octyloxy.

More specific peptides within the scope of the Invention and having this general formula are shown in Table 1.

**Table 1**

| Compound No. | Compound Name |
|---|---|
| 1 | H-Gly-Lys(4-nitrobenzoyl)-OH |
| 2 | H-Gly-Lys(4-fluorobenzoyl)-OH |
| 3 | H-Gly-Lys(4-cyanobenzoyl)-OH |
| 4 | H-Gly-Lys(4-methoxybenzoyl)-OH |
| 5 | H-Gly-Lys(4-chlorobenzoyl)-OH |
| 6 | H-Gly-Lys(benzoyl)-OH |
| 7 | H-Lys(4-nitrobenzoyl)-Gly-OH |
| 8 | H-Lys(4-nitrobenzoyl)-Sar-OH |
| 9 | H-Lys(benzoyl)-Sar-OH |
| 10 | H-Lys(benzoyl)-Gly-OH |
| 11 | H-Lys(4-methoxybenzoyl)-Gly-OH |
| 12 | H-Gly-D-Lys(4-methoxybenzoyl)-OH |
| 13 | H-Gly-D-Lys(4-nitrobenzoyl)-OH |
| 14 | H-Gly-D-Lys(4-fluorobenzoyl)-OH |
| 15 | H-Gly-D-Lys(4-cyanobenzoyl)-OH |
| 16 | H-Gly-D-Lys(4-nitrobenzoyl)-OH |
| 17 | H-Gly-D-Lys(benzoyl)-OH |
| 18 | H-Lys(4-fluorobenzoyl)-Gly-OH |
| 19 | H-Lys(4-cyanobenzoyl)-Gly-OH |
| 20 | H-Lys(4-chlorobenzoyl)-Gly-OH |
| 21 | H-D-Lys(4-methoxybenzoyl)-Gly-OH |
| 22 | H-D-Lys(4-nitrobenzoyl)-Gly-OH |
| 23 | H-D-Lys(benzoyl)-Gly-OH |
| 24 | H-D-Lys(4-fuorobenzoyl)-Gly-OH |
| 25 | H-D-Lys(4-cyanobenzoyl)-Gly-OH |
| 26 | H-D-Lys(4-chlorobenzoyl)-Gly-OH |
| 27 | H-Lys(4-cyanobenzoyl)-Sar-OH |
| 28 | H-Lys(4-methoxybenzoyl)-Sar-OH |
| 29 | H-Lys(4-fuorobenzoyl)-Sar-OH |
| 30 | H-Lys(4-chlorobenzoyl)-Sar-OH |
| 31 | H-D-Lys(4-cyanobenzoyl)-Sar-OH |
| 32 | H-D-Lys(4-methoxybenzoyl)-Sar-OH |
| 33 | H-D-Lys(4-fuorobenzoyl)-Sar-OH |
| 34 | H-D-Lys(4-chlorobenzoyl)-Sar-OH |
| 35 | H-D-Lys(4-nitrobenzoyl)-Sar-OH |
| 36 | H-D-Lys(benzoyl)-Sar-OH |
| 37 | H-Ala-D-Lys(4-methoxybenzoyl)-OH |

(continued)

Table 1

| Compound No. | Compound Name |
|---|---|
| 38 | H-Val-D-Lys(4-methoxybenzoyl)-OH |
| 39 | H-Ile-D-Lys(4-methoxybenzoyl)-OH |
| 40 | H-Leu-D-Lys(4-methoxybenzoyl)-OH |
| 41 | H-Phe-D-Lys(4-methoxybenzoyl)-OH |
| 42 | H-Trp-D-Lys(4-methoxybenzoyl)-OH |
| 43 | H-His-D-Lys(4-methoxybenzoyl)-OH |
| 44 | H-Tyr-D-Lys(4-methoxybenzoyl)-OH |
| 45 | H-D-Lys(4-methoxybenzoyl)-Ala-OH |
| 46 | H-D-Lys(4-methoxybenzoyl)-Phe-OH |
| 47 | H-D-Lys(4-methoxybenzoyl)-Ile-OH |
| 48 | H-D-Lys(4-methoxybenzoyl)-Leu-OH |
| 49 | H-D-Lys(4-methoxybenzoyl)-Val-OH |
| 50 | H-D-Lys(4-methoxybenzoyl)-His-OH |
| 51 | H-D-Lys(4-methoxybenzoyl)-Trp-OH |
| 52 | H-D-Lys(4-methoxybenzoyl)-Tyr-OH |
| 53 | H-D-Lys(4-phenoxybenzoyl)-Gly-OH |
| 54 | H-D-Lys(4-t-butylbenzoyl)-Gly-OH |
| 55 | H-D-Lys(4-n-butoxybenzoyl)-Gly-OH |
| 56 | H-D-Lys(4-methylbenzoyl)-Gly-OH |
| 57 | H-D-Lys(4-ethylbenzoyl)-Gly-OH |
| 58 | H-D-Lys(4-n-butylbenzoyl)-Gly-OH |
| 59 | H-D-Lys(4-n-hexylbenzoyl)-Gly-OH |
| 60 | H-D-Lys(4-n-octylbenzoyl)-Gly-OH |
| 61 | H-D-Lys(4-pheylbenzoyl)-Gly-OH |
| 62 | H-D-Lys(4-benzyloxybenzoyl)-Gly-OH |
| 63 | H-D-Lys(4-ethoxybenzoyl)-Gly-OH |
| 64 | H-Asn(NH(4-trifluoromethylbenzyl))-Ala-OH |
| 65 | H-Asn(NH(4-methoxybenzyl))-Ala-OH |
| 66 | H-Asn(NH(4-nitrobenzyl))-Ala-OH |
| 67 | H-Asn(NH(benzyl))-Ala-OH |
| 68 | H-Asn(NH(4-fluorobenzyl))-Ala-OH |
| 69 | H-Asn(NH(4-chlorobenzyl))-Ala-OH |
| 70 | H-Asn(NH(4-cyanobenzyl))-Ala-OH |
| 71 | H-Asn(NH(4-methylbenzyl))-Ala-OH |
| 72 | H-Asn(NH(4-n-butylbenzyl))-Ala-OH |
| 73 | H-Asn(NH(4-t-butylbenzyl))-Ala-OH |
| 74 | H-Asn(NH(4-n-hexylbenzyl))-Ala-OH |
| 75 | H-Asn(NH(4-n-octylbenzyl))-Ala-OH |
| 76 | H-Asn(NH(4-phenylbenzyl))-Ala-OH |
| 77 | H-Asn(NH(4-phenoxybenzyl))-Ala-OH |
| 78 | H-Asn(NH(4-n-butoxybenzyl))-Ala-OH |
| 79 | H-Asn(NH(4-trifluoromethylbenzyl))-D-Ala-OH |
| 80 | H-Asn(NH(4-methoxybenzyl))-D-Ala-OH |
| 81 | H-Asn(NH(4-nitrobenzyl))-D-Ala-OH |
| 82 | H-Asn(NH(benzyl))-D-Ala-OH |
| 83 | H-Asn(NH(4-fluorobenzyl))-D-Ala-OH |
| 84 | H-Asn(NH(4-chlorobenzyl))-D-Ala-OH |
| 85 | H-Asn(NH(4-cyanobenzyl))-D-Ala-OH |

(continued)

Table 1

| Compound No. | Compound Name |
|---|---|
| 86 | H-Asn(NH(4-methylbenzyl))-D-Ala-OH |
| 87 | H-Asn(NH(4-n-butylbenzyl))-D-Ala-OH |
| 88 | H-Asn(NH(4-t-butylbenzyl))-D-Ala-OH |
| 89 | H-Asn(NH(4-n-hexylbenzyl))-D-Ala-OH |
| 90 | H-Asn(NH(4-n-octylbenzyl))-D-Ala-OH |
| 91 | H-Asn(NH(4-phenylbenzyl))-D-Ala-OH |
| 92 | H-Asn(NH(4-phenoxybenzyl))-D-Ala-OH |
| 93 | H-Asn(NH(4-n-butoxybenzyl))-D-Ala-OH |
| 94 | H-D-Asn(NH(4-trifluoromethylbenzyl))-Ala-OH |
| 95 | H-D-Asn(NH(4-methoxybenzyl))-Ala-OH |
| 96 | H-D-Asn(NH(4-nitrobenzyl))-Ala-OH |
| 97 | H-Asn(NH(4-methoxybenzyl))-Sar-OH |
| 98 | H-Asn(NH(4-methoxybenzyl))-Leu-OH |
| 99 | H-Asn(NH(4-methoxybenzyl))-Phe-OH |
| 100 | H-Gln(NH(4-methoxybenzyl))-Ala-OH |
| 101 | H-Orn(4-methoxybenzoyl)-Gly-OH |
| 102 | H-Gly-Asn(NH(4-methoxybenzyl))-OH |
| 103 | H-D-Lys(2,4-dinitrobenzoyl)-Gly-OH |
| 104 | H-D-Lys(2,4-dimethylbenzoyl)-Gly-OH |
| 105 | H-D-Lys(2,5-dimethylbenzoyl)-Gly-OH |
| 106 | H-D-Lys(3,5-dimethylbenzoyl)-Gly-OH |
| 107 | H-D-Lys(2,4-dichlorobenzoyl)-Gly-OH |
| 108 | H-D-Lys(2,5-dichlorobenzoyl)-Gly-OH |
| 109 | H-D-Lys(4-fluoro-3-nitrobenzoyl)-Gly-OH |
| 110 | H-D-Lys(3-fluoro-4-methylbenzoyl)-Gly-OH |

More particular peptides according to the invention in one aspect facilitate and/or maintain or inhibit the intercellular communication mediated by gap junctions. In one embodiment the peptides may be antiarrhythmic peptides which target the same cells targeted by AAP, AAP10, HP5, and/or functional analogs thereof, i.e., the peptides are able to modulate the function of these cells by agonizing or antagonizing the function of AAP, AAP10, HP5, and/or functional analogs thereof. The invention also relates to the preparation and use of pharmaceutical compositions for the treatment of pathologies associated with impaired intercellular gap junctional communication and methods for using these compositions.

Further preferred peptides in accord with the invention show good activity in one or more of the following assays. Although not necessary to identify orally available peptides as represented by Formulae I and II, above, they can be used to further confirm and optionally quantify activity of one or a pool of peptides.

In one embodiment of the invention the peptide is selected from the group consisting of H-Gly-Lys(4-nitrobenzoyl)-OH (Compound 1); H-Gly-Lys(4-methoxybenzoyl)-OH (Compound 4); H-D-Lys(4-methoxybenzoyl)-Gly-OH (Compound 21); H-$_D$-Lys(4-nitrobenzoyl)Gly-OH (Compound 22); H-D-Lys(4-t-butylbenzoyl)-Gly-OH (Compound 54); and H-$_D$-Asn(NH(4-nitrobenzyl)Ala-OH (Compound 96).

In another embodiment the present peptide is selected from the group consisting of H-$_D$-Lys(benzoyl)Gly-OH (Compound 23) and H-$_D$-Asn(NH(4-methoxybenzyl)Ala-OH (Compound 95).

In yet another embodiment of the invention the peptide is H-Gly-Lys(4-nitrobenzoyl)-OH (Compound 1).

According to the invention in one aspect the peptide is H-Gly-Lys(4-methoxybenzoyl)-OH (Compound 4).

Further, in yet another embodiment the peptide is H-D-Lys(4-methoxybenzoyl)-Gly-OH (Compound 21).

In yet another aspect the peptide is H-$_D$-Lys(4-nitrobenzoyl)Gly-OH (Compound 22).

In a further aspect the peptide is H-D-Lys(4-t-butylbenzoyl)-Gly-OH (Compound 54).

In still another aspect the peptide is H-$_D$-Asn(NH(4-nitrobenzyl)Ala-OH (Compound 96).

Further, in yet another embodiment the peptide peptide is H-$_D$-Lys(benzoyl)Gly-OH (Compound 23).

In still another embodiment the peptide is H-$_D$-Asn(NH(4-methoxybenzyl)Ala-OH (Compound 95).

Preferred peptides according to Formulae I and II above show good function as a modulator of gap junctional communication (e.g., as agonists or antagonists). In one aspect the peptides have the function as an antiarrhythmic drug.

In one embodiment the preferred agonist peptides of the invention provide an intracellular conductance (Gj) that is substantially the same as, or is greater than, the Gj of AAP in what is referred to herein as a "standard cardiomyocyte assay".

In another embodiment the preferred antagonist peptides provide a Gj that is less than the Gj of AAP and/or block the ability of AAP to normalize the Gj of an ischemic cell, i.e., to return the Gj to substantially the same values found in non-ischemic cells. In one aspect of the invention the present antagonists provide a Gj that is at least about 40% less than the Gj of AAP, such as at least about 50% less, for example at least about 60% less, such as at least about 70% less, for example at least about 80% less, such as at least 90% less, for example at least 100% less.

The inhibitory properties of the present antagonists are not limited to the relative Gj as described above. In further embodiments and assay types the antagonists may display a different relative Gj. This may further depend on the comparative peptide/compound.

Heart

Additionally preferred peptides according to the invention increase the time to an AV block in a mouse after infusion of $CaCl_2$, in what is referred to herein as a "standard calcium-induced arrhythimia assay". Preferably, the peptides provide at least about 40% of the activity of AAP, for example at least about 50% of the activity of AAP, such as about 60% of the activity of AAP, for example at least about 70% of the activity of AAP, such as at least about 80% of the activity of AAP, for example at least about 90% of the activity of AAP, for example at least about substantially the same activity of AAP, such as about 110% of the activity of AAP, for example at least about 120% of the activity of AAP, such as at least about 130% of the activity of AAP, for example at least about 140% of the activity of AAP, such as about 150% of the activity of AAP, for example at least about 160% of the activity of AAP, such as at least about 170% of the activity of AAP, for example at least about 180% of the activity of AAP, preferably at least about 190% of the activity of AAP, more preferably at least about 200 or greater % of the activity of AAP (i.e., show time lags of approximately the same duration).

Peptides may additionally show decreases in the incidence of reentry arrhythmias or in the size of an infarct zone observed in what is referred to herein as a "standard ventricular reentry assay". Preferably, the peptides provide at least about 40% of the activity of AAP, for example at least about 50% of the activity of AAP, such as about 60% of the activity of AAP, for example at least about 70% of the activity of AAP, such as at least about 80% of the activity of AAP, for example at least about 90% of the activity of AAP, for example at least about substantially the same activity of AAP, such as about 110% of the activity of AAP, for example at least about 120% of the activity of AAP, such as at least about 130% of the activity of AAP, for example at least about 140% of the activity of AAP, such as about 150% of the activity of AAP, for example at least about 160% of the activity of AAP, such as at least about 170% of the activity of AAP, for example at least about 180% of the activity of AAP, preferably at least about 190% of the activity of AAP, more preferably at least about 200 or greater % of the activity of AAP (i.e., providing similar decreases in incidence or infarct zones of similar or smaller size).

Osteoporosis

There is understanding that GJIC is important in bone formation. Additionally preferred peptides additionally, or alternatively, increase osteoblast activity in what is referred to herein as a "standard osteoblast activity assay" which measures either calcium wave formation and/or alkaline phosphatase activity of osteoblast cells in the presence of peptides. Preferably, such peptides increased calcium wave activity, manifested as an increase in the number of cells involved in a wave (as determined by measuring levels of intracellular $Ca^{2+}$ using a calcium sensitive fluorescent dye, such as fura-2 and counting the number of cells which fluoresce). Alkaline phosphatase activity also can be used to provide a measure of osteoblast activity using standard colorimetric assays. Agonist peptides according to the invention provide at least about 10% of the activity of AAP in such an assay, such as at least about 20% activity, for example at least about 30% activity, such as at least about 40% activity, for example at least about 50% of the activity of AAP, preferably, at least about 70% activity, and still more preferably, 100% or greater activity of the activity of AAP.

Cancer

Preferred peptides according to the invention, alternatively, or additionally, decrease GJIC inhibition mediated by tumor promoters such as DTT, in what is referred to herein as a "standard tumor promoter assay." Preferably, the peptides show decreases in GJIC inhibition, which are at least 50%, preferably 70%, and more preferably 100% or greater, than decreases observed for AAP.

As discussed, it is an object of the invention to provide peptides that modulate gap junction intercellular communication (GJIC). Thus, many peptides in accord with the invention may include one or more of the following features: the ability to decrease cellular uncoupling, to normalize dispersion of action potential duration, and to normalize conduction velocity, the ability to control of the cellular quantity of gap junctions normalizing (up-regulating or down-regulating as needed) the expression of connexins; to normalize degradation of gap junctions (inhibit or enhance), to normalize cellular trafficking of connexins to the plasma membrane (increase or decrease); to facilitate assembly of connexins into functional gap junctions; to normalize opening of existing gap junctions, e.g., inducing or enhancing opening when they have been closed or gated by inhibitors (e.g., such as by mediating or enhancing hyperphosphorylation of the cytoplasmic carboxy terminal domain of one or more connexins (e.g., such as Cx43)) or closing these when they are aberrantly opened (e.g., as in Charcot-Marie-Tooth disease); and the like.

Particular assays useful for identifying and optionally quantifying the activity of preferred invention peptides are described below. The assays are non-limiting and are merely serving the purpose of illustrating a variety of assays in which the present peptides may be tested for their gap junction modulating abilities. It is to be understood that the assays are not mutually exclusive, i.e. a peptide according to the invention may show activity in one particular assay, but show a different, or no, activity in another particular assay. This may be a reflection of the diversity of the individual peptides and types of assays for testing said peptides.

**A. Standard Oral Availability Assay**

It is a preferred aspect of the invention to provide peptides with enhanced availability *in vivo.* Absorption of peptides after oral administration is often limited because they are degraded by either enzymes in the gastrointestinal (GI) tract or by enzymes in the intracellular lumen of the enterocytes. Further, the physico-chemical properties of peptides, especially their large hydrogen-bonding potential, makes it difficult for these molecules to permeate the enterocytes by transcellular passive diffusion. However, di- and tri-peptides, which have survived the gastrointestinal fluid enzymes can enter the intracellular lumen by means of the peptide transporter hPepT1, which is a membrane protein localized in the apical membrane of the enterocytes of the upper small intestine.

Preferred peptides according to the invention are therefore peptides that have affinity for an hPepT1 transporter or an analog thereof. The three-dimensional conformation and key binding sites of peptide compounds which bind the PepT1 transporter are described by Bailey, P.D., et al., 2000, *supra,* and desired peptides can be modeled *in silico* to optimally fit within this binding site, as described above (see, e.g., Bailey, P.D., et al., (2000), *supra*; Vinter, J.G., (1996) J. Comput. Aided Des. 10: 417).

The oral availability of a peptide comprising a structure as shown in Formula I, Formula II, Table 1, or more generally identified using the Bailey assay, may be evaluated for its ability to bind to a PepT1 transporter, preferably an hPepT1 transporter. For example, a PepT1 cDNA, preferably, an hPepT1 cDNA (see, e.g., Covitz, K. M., et al., (1996) Pharm. Res. 13(11): 1631-34) may be expressed in a *Xenopus* oocyte expression system and the uptake of labeled peptide into the oocyte can be monitored to approximate Ki values as described in Temple, C.S., et al. (1996) J. Physiol. (London) 494: 795; Meredith, D., et al., (1998) J. Physiol. (London) 512: 629.

In one aspect of the invention, a standard *in vivo* oral availability assay is performed to determine the oral availability of a peptide which has been modeled to optimally conform to the Bailey substrate template, as discussed above. In this assay, a peptide is orally administered to a mammal, such as a rat, in anorally administrable form (e.g., as part of a food pellet or in water), while at the same time, the same concentration of peptide is administered i.v. (e.g., through a catheter inserted into the femoral vein and artery). Peptides may be administered as bolus injections at concentrations ranging from $10^{-5}$-$10^{-10}$ in volumes of 1 ml/kg for both oral and i.v. dosing. The animals are given 500 I.U. of heparin i.v. 5 minutes before the first blood sample is taken. A control blood sample, "before dose" or B.D. sample, is collected approximately 5 minutes before administration of the peptides. A sample of the dosing solution (e.g., 100 μl of water comprising $10^{-5}$-$10^{-10}$ M of peptide) is retained for concentration determination. Blood samples are collected at t=B.D. 5, 15, 30, 60, 90, 120, 180, and 240 minutes.

Blood is collected in labeled ice-chilled EDTA stabilized blood sample vials and stored on ice until quickly centrifuged at 4˚C for 5 minutes (10,000 x g). Plasma (100 μl) is harvested, transferred to a labeled polypropylene microcentrifuge vial (e.g., 0.5 ml eppendorf), frozen on ice and stored at -20˚C until further analysis. Approximately 40 μl of the filtrate is injected onto an HPLC column (XterraMS C18, 3 x 50 mm, 3.5 μm particles) and eluted using a linear gradient from 0 to 100% B in 4.0 minutes. The column is washed for 2.9 minutes in buffer B (0.1% formic acid in acetonitrile or another suitable buffer) and equilibrated for 5 minutes in Buffer A (0.1 % formic acid in water or another suitable buffer) prior to the next injection of sample. Mass spectrometry is performed using methods routine in the art and as described further below in Examples 1 and 2.

The concentrations of compounds in the plasma samples are calculated from an external standard curve covering the range from 1.00 to 1000 nM. The plasma concentrations versus time data are used for pharmacokinetic modeling in WinNonLin 3.5 (Pharsight, Mountain view, CA) using non-compartmental analysis and AUC values are

determined as is known in the art. Preferably, orally available peptides according to the invention are observed in significant levels In plasma within about 30 minutes or less. AUC values observed in animals receiving i.v. administrations of peptide are used to evaluate such effects as clearance and half-life which should be the same in the two systems.

### B. Standard Plasma Stability Assays

The invention also provides peptides that have enhanced stability *In vitro* or *In vivo.* In one aspect the peptide comprises a peptide bond that is alkylated or otherwise modified to stabilize the peptide against enzymatic degradation. In another aspect, the peptide comprises one or more D-amino acids. In a further aspect, the peptide has enhanced stability in a standard stability assay.

In one aspect, an *in vitro* plasma stability assay is performed as described in WO 02/077017 (PCT/US02/05773, filed February 22, 2002).

As disclosed in WO02/077017, peptides can be incubated in plasma or serum and samples taken at regular intervals for analysis by HPLC or LC/MS/MS, to quantitate the amount of undegraded peptide. Appropriate conditions (column, solvent, gradient, and temperature) for such analyses are estimated to ensure that the peptide peak and the plasma peaks do not have the same retention time. This is done by subsequent Injections of a peptide, plasma, and a co-injection with the peptide and the plasma, followed by optimization of LC method parameters until a satisfactory separation is obtained. A control plasma sample without the peptide, treated in the same manner, also can be taken and evaluated. The samples may include, but are not limited to, a blank, the peptide at a suitable concentration (e.g., 0,1 mg/mL), plasma without peptide, one or more samples for t = 0, and one or more samples at each regular interval. Preferably, multiple samples are taken in parallel. The sample concentrations (peak height in mAU or ion counts) can be plotted vs, time and fitted to a function describing a mono exponential decay (e.g., using a standard Excel package). Preferably, a peptide according to the invention has a half-life of more than about 48 hours, such as more than 24 hours, for example more than 12 hours, such as more than 6 hours, for example more than 3 hours, such as more than 1 hour, for example more than 30 minutes as determined using this assay.

Plasma stability can be examined *in vivo* using standard assays. For example, peptides may be administered to a mammal, such as a rat, by bolus injections in volumes of about 1 ml/kg for both i.v. and p.o. dosing. Preferably, peptides are tested in parallel with control samples such as buffer or an antiarrythmic peptide with a known stability. Blood samples are collected at different time periods (e.g., at B.D. 5, 15, 30, 60, 90, 120, 180, and 240 minutes, where B.D. refers to before dose). Amounts of peptides in samples can be quantitated using methods routine in the art, such as LC/MS/MS. For example, the concentrations of peptides in plasma samples may be calculated from an external standard curve covering ranges of peptide from 1.00 to 1000 nM. The plasma concentrations versus time data can be used for pharmacokinetic modeling in WinNonLin 3.5 (Pharsight, Mountain view, CA) using non-compartmental analysis and the resulting parameters of AUC, Fpo, Clb, t1/2, Cmax and tmax can be determined as is known in the art.

### C. Standard Cardiomyocyte Assays

In one aspect, a peptide according to the invention is administered to a cardiac cell and gap junction function is evaluated. Optimal peptides for such procedures can be identified in standard cardiomycte assays. In one aspect, cardiac cells are isolated from a mammal, such as a guinea pig hearts by perfusion with collagenase according to the Langendorf method. The cells are exposed to peptide and evaluated for GJIC by patch clamp using methods known in the art. Intercellular conductance (Gj) using the formula:

$$G_j = \frac{\Delta I_p}{\Delta U_j} = \frac{I_{p,pulse} - I_{p,rest}}{U_p - U_a} \quad \text{(Equation 1)}$$

Where Ip,pulse and Ip,rest represent the current in the passive cell during the pulse and before the pulse respectively, and Up and Ua represent the voltage of the passive and active cell. The change in Gj value upon peptide administration is analyzed by comparing the relative changes in Gj. For example, the relative Gj as a function of time before, and during, stimulation with peptide (e.g., at about $10^{-8}$ M) can be determined.

In one embodiment the peptide provides a Gj, which is substantially the same as the Gj (by ± 10%) of an antiarrhythmic peptide such as AAP, AAP10, HP5, and functional analogs thereof. In one aspect, the cell is an ischemic cell, and the peptide provides a Gj, which is substantially the same as that of a non-ischemic cell (±20%, preferably, ± 10%).

In another embodiment the present peptides provides a GJ, which is different from the Gj (by ± 40%) of an

antiarrhytmic peptide such as AAP, AAP10, HP5, and functional analogs thereof.

Additional details concerning performing cardiomyocyte assays are provided in WO2002/077017 (PCT/US02/05773, filed February 22, 2002).

### D. Standard Caiclum-induced Arrhythmia Assay

Peptides suitable for administration to cardiac cells can be identified in an *in vivo* model of calcium-induced arrhythmias according to the model of Lynch et al. (1981) J Cardiovasc.Pharmacol. 3; 49-60. Mice (25-30 g) are anaesthetized with a neurolept anaesthetic combination and an i.v. cannula is inserted into the tail vein. A lead II ECG signal is recorded continuously by positioning a stainless steel ECG electrodes on the right forelimb and on the left hind limb. The ground electrode is placed on the right hind limb. The signal is amplified (x 5.000-10.000) and filtered (0.1-150 Hz) via a Hugo Sachs Electronic model 689 ECG module. The analog signal Is digitized via a 12 bit data acquisition board (Data Translation model DT321) and sampled at 1000 Hz using the Notocord HEM 3.1 software for Windows NT. After a 10-minute equilibration period, the test sample of peptide is injected into the tail vein. Mice pre-treated with buffer are tested as a measure of the control level In untreated animals. The injection volume is 100 μl in all experiments.

Infusion of $CaCl_2$ (30 mg/ml, 0.1 ml/min ≈ 100 mg/kg/min (calcium chloride-2-hydrate, Riedel-de Haën, Germany)) is started 3 min after i.v. administration of drug or vehicle. The time lag to onset of 2nd degree AV-block is determined as the time from the start of CaCl2 infusion until the first arrhythmic event occurs. An event of 2nd degree AV-block Is defined as intermittent failure of the AV conduction characterised by a P-wave without the concomitant QRS complex.

Responses are expressed relative to the time until 2nd degree AV-block occurred In vehicle treated mice. The maximal effect of compounds (e.g., peptides, AAP, AAP10 or controls) is determined. Preferably, peptides according to the invention have antiarrhythmic activity comparable to the compounds AAP, AAP10, HP5, or a functional analog thereof, i.e., the peptides increase the time to an AV block in a mouse after Infusion of $CaCl_2$. Preferably, Preferably, the peptides provide at least about 40% of the activity of AAP, for example at least about 50% of the activity of AAP, such as about 60% of the activity of AAP, for example at least about 70% of the activity of AAP, such as at least about 80% of the activity of AAP, for example at least about 90% of the activity of AAP, for example at least about substantially the same activity of AAP, such as about 110% of the activity of AAP, for example at least about 120% of the activity of AAP, such as at least about 130% of the activity of AAP, for example at least about 140% of the activity of AAP, such as about 150% of the activity of AAP, for example at least about 160% of the activity of AAP, such as at least about 170% of the activity of AAP, for example at least about 180% of the activity of AAP, preferably at least about 190% of the activity of AAP, more preferably at least about 200 or greater % of the activity of AAP (i.e., the peptides show time lags of approximately the same duration as induced by AAP).

### E. Standard Osteoblast Activity Assay

Modulation of intercellular communication represents a mechanism by which osteotropic factors regulate the activity of bone forming cells. Therefore, in one aspect, peptides according to the invention are used to increase osteoblast activity by increasing gap junctional communication between bone cells, thereby enhancing bone formation *in vivo.*

The efficacy of a peptide according to the invention may be assayed in preliminarily in human osteblast cells (hOB), for example by measuring calcium wave activity and/or alkaline phosphatase activity.

In one aspect, cells are isolated from human bone marrow obtained by puncture of the posterior iliac spine of healthy volunteers (aged 20-36): 10-15 ml marrow material was collected in 15 ml PBS+Ca,Mg (Life Technologies, Cat. No. 14040) with 100 U/ml Heparin (Sigma, Cat. No. H-3149). The mononuclear fraction of the marrow is isolated on a Lymphoprep gradient (Nycomed Pharma, Cat.No. 1001967), by centrifugation at 2200 rpm for 30 min. After harvesting, the mononuclear fraction is washed once with culture medium and centrifuged at 1800 rpm for 10 min. Subsequently cells are counted and plated in culture medium at $5x10^6$ cells/100 mm dish. hOB medium (all reagents obtained from Life Technologies): MEM w/o Phenol Red w/ Glutamax (Cat.No. 041-93013) supplemented with 10% heat inactivated fetal calf serum (Cat.No. 10106) and 0.1% Penicillin/Streptomycin (Cat.No. 15140). Medium is changed the following day and the cells are cultured at 37˚C in 5%$CO_2$ with medium change every 7 days. After 4-6 weeks of culture, the cells will reach 70% confluence. The medium is then supplemented with 100 nM Dexamethasone (Sigma, Cat.No. D-4902) for 7 days. Cells are then plated for video imaging experiments: a 25 mm #1 glass coverslip is placed in a 35 mm dish (or each well of a 6-well multidish), cells are plated at $2.5x10^5$ cells/coverslip and cultured for 2-3 days before use.

ROS 17/2.8 cells are cultured in 100 mm dishes at 37˚C with 5% $CO_2$ and medium changed every 2-3 days. ROS medium (all reagents obtained from Life Technologies): MEM (Cat.No. 31095) is supplemented with 10% heat-inactivated calf serum (Cat.No. 16170), 1% NEAA (Cat.No. 11140), 1% Sodium Pyruvate (Cat.No. 11360), 1% L-Glutamine (Cat.No. 25030) and 0.1% Penicillin/Streptomycin (Cat.No. 15140). For video imaging experiments, cells are plated on coverslips at $2-3x10^5$ cells/coverslip and cultured for 2-3 days before use.

The cells cultured on coverslips are loaded with 5 μM fura-2-AM (Molecular Probes, Cat.No. F-1221), for 30 minutes at 37°C, and incubated in fresh medium for 20 minutes. Coverslips are then affixed to a PDMI-2 culture chamber (Medical Systems Corp.), maintained at 37°C with superfused $CO_2$, on a Zeiss Axiovert microscope. Intercellular calcium waves are induced by mechanical stimulation of a single cell using a borosilicate glass micro pipette affixed to an Eppendorf 5171 micromanipulator.

Imaging is performed using a MetaMorph imaging system (Universal Imaging). The excitation light (340 and 380 nm) is provided by a monochromator (T.I.L.L. Photonics GmbH). Images are acquired with an intensified CCD camera (Dage MTI) and digitized with a Matrox MVP image processing board. The number of cells involved in a calcium wave in the presence and absence of peptide can be used to provide a measure of increase in GJIC.

In one aspect, administration of a peptide increases the number of cells involved in a wave at least about two-fold compared to cells which have been exposed to a control, such as buffer. In another aspect, administration of a peptide decreases the number of cells involved in a wave by at least about two-fold. Agonist peptides according to the invention provide at least about 10% of the activity of AAP in such an assay, such as at least about 20% activity, for example at least about 30% activity, such as at least about 40% activity, for example at least about 50% of the activity of AAP, preferably, at least about 70% activity, and still more preferably, 100% or greater activity of the activity of AAP.

Cells also can be measured for the presence of alkaline phosphatase activity to provide a general measure of osteoblast activity. In one aspect, cells are plated in 96-well plates at a concentration of 8000 cells/well (hOB) or 3000 cells/well (ROS) in 200μl normal culture medium. On day 4 (or day 3 for ROS cells), cells are washed with 200 μl MEM, 0.1% BSA (Sigma, Cat.No. A-9418). Samples comprising a suitable medium (e.g., 200 μl MEM, 0.1% BSA) containing various concentrations of peptides, control, AAP or AAP10 are added to the cells, and culture is continued for about 4 days (2 days for ROS cells).

On about day 8 (preferably day 5 for ROS cells), cells are assayed for alkaline phosphatase using an Alkaline Phosphatase (ALP) assay such as is known in the art. ALP assays are generally colorimetric endpoint methods for measuring enzyme activity, and can be performed using an Alkaline Phosphatase Kit (Sigma, Cat.No. 104-LL). Preferably, cells are washed once with 200 μl PBS+Ca, Mg, 100μl Alkaline Buffer Solution is added to each well and the cells are incubated at 37°C for 10 minutes. 100 μl Substrate Solution is subsequently added to each well and the plate is incubated at 37°C for 30 min. 100 μl 2.0 N NaOH is added to each well to stop the reaction. Absorbance is measured using a plate reader at 405 nm.

Agonist peptides according to the invention provide at least about a 5% increase in alkaline phosphatase production relative to isotonic saline, preferably, at least about 10% increase in alkaline phosphatase production relative to isotonic saline, and still more preferably a 15% or greater increase in alkaline phosphatase production relative to isotonic saline. The increase in production of alkaline phosphatase is a measure for increased activity of osteoblasts and accordingly a measure for an increase in bone formation.

**F. Standard Tumor Promotor Assay**

The compound 1,1-bis(*p*-chlorophenyl)-2,2,2-trichlorethane, also known as the insecticide DDT, is an inhibitor of gap junctional communication, and has tumor promoting abilities. It inhibits cell-to-cell communication by reducing the gap junction number and size, and exposure to DDT is associated with decreased cellular levels of phosphorylated (active) forms of the gap junction protein Cx43. These actions are considered pivotal for the compound's oncogenic properties (X. Guan, et al. (1996) Carcinogenesis, 17 1791-1798; R. J. Ruch, et al. (1994) Carcinogenesis, 15 301-306); B. V. Madhukar, et al. (1996) Cancer Lett. 106 117-123). As a means of monitoring the therapeutic efficacy of the peptides, the effects of the peptides on DDT-induced uncoupling in human osteoblast cells can be determined. Thus, in one aspect, peptides according to the invention are used to inhibit or prevent tumor-promoter induced decreases of GJIC (W. K. Hong, et al. (1997) Science, 278 1073-1077).

In one exemplary assay, human osteoblast cells are isolated from human bone marrow obtained by puncture of the posterior iliac spine of healthy volunteers (aged 20-36). Approximately 10-15 ml of bone marrow material are collected in 15 ml PBS + Ca, Mg (Life Technologies, Cat.No. 14040) with 100 U/ml Heparin (Sigma, Cat.No. H-3149). The mononuclear fraction of the marrow is isolated on a Lymphoprep gradient (Nycomed Pharma, Cat.No. 1001967), by centrifugation at 2200 rpm for 30 minutes.

After harvesting, the mononuclear fraction iswashed once with culture medium and centrifuged at 1800 rpm for 10 minutes. Subsequently cells are counted and plated in culture medium at $8x10^6$ cells/100 mm dish. Medium is changed the following day and the cells are cultured at 37°C in 5%$CO_2$ with medium changes every 7 days. After 3-4 weeks of culture, the cells typically reach 70% confluence. The medium is then supplemented with 100 nM Dexamethasone (Sigma, Cat.No. D-4902) for 7 days. Cells are then plated for video imaging experiments. Generally, cells are plated at $2.5x10^5$ cells/coverslip and are cultured for 2-3 days before imaging.

After culturing, cells are affixed to a PDMI-2 culture chamber (Medical Systems Corp.), maintained at 37°C with superfused $CO_2$, on a Zeiss Axiovert microscope. Microinjections are performed using a micropipette is loaded with

a 10 mM Lucifer Yellow solution (Sigma, Cat.No. L-0259). A cell in the monolayer is carefully injected with LY for 30 seconds; the micropipette is removed from the cell and after 30 seconds the number of cells that show dye transfer is counted. For a subset of cell cultures, DDT is added to the medium in a final concentration of 13 μM, and is left on for 60 minutes. Images of cells are acquired with an intensified CCD camera (Dage MTI) and digitized with a Matrox MVP image processing board, using the MetaMorph imaging software (Universal Imaging).

Under control conditions (no DDT treatment), the dye generally spreads to a median of 14.5 cells (n=12). DDT-exposed cells typically show decreased cellular coupling with a median of 7 (n=13).

Peptides are added to the bathing solution in a final concentration of $10^{-8}$ mol/l, and after 10 minutes, another microinjection is performed. Preferably, agonist peptides according to the invention show an increase in cell-to-cell dye transfer. More preferably, this increase is significantly different from control samples (without peptides) as determined using routine statistical tests, such as the Wilcoxon non-parametric statistical test (with $p<0.05$). Preferably, the peptides show decreases in GJIC inhibition which are at least about 50%, preferably about 70%, and more preferably, about 100% or greater, than decreases observed for AAP.

This assay can be used to identify candidate peptides with the highest therapeutic efficacy in reversing the decreased intercellular coupling related to tumor promotion and in one aspect, such peptides are administered to patients at risk for developing or having cancer. A peptide may be used alone or in combination with other peptides and/or in a combination therapy with other anti-cancer agents.

Still other assays may be performed to identify peptides which elicit substantially the same physiological responses as the antiarrhythmic peptides AAP, AAP10, HP, and their functional analogs (e.g., to identify agonists) or which inhibit or suppress these physiological responses (e.g., to identify antagonists). Suitable assays include, but are not limited to: assays to measure cAMP formation in cells (e.g., CHO cells); cAMP efficacy assays (e.g., measuring inhibition of forskoline-stimulated cAMP formation of APP-like compounds in CHO cells); phosphoinositol turnover in cardiomyocytes (Meier et al.) (E. Meier, et al. (1997) Drug Development Research, 40: 1-16); and responses to glucose and oxygen deprivation.

A number of standard assays are detailed above. Additional assays are described in PCT/US02/05773, filed February 22, 2002, the entirety of which is incorporated herein by reference. These assays are exemplary only and other suitable assays that may be developed and become standardized are encompassed within the scope of the invention.

## Pharmaceutical Compositions

The peptides may be formulated in a pharmaceutical composition comprising one or more of any of the peptides described above, in combination with a pharmaceutically acceptable carrier and/or diluent. Such compositions are preferably in a form adapted for oral administration.

Formulations for oral administration may be prepared in a manner well-known to the person skilled in the art, e.g., as generally described in "Remington's Pharmaceutical Sciences", 17. Ed. Alfonso R. Gennaro (Ed.), Mark Publishing Company, Easton, PA, U.S.A., 1985 and more recent editions and in the monographs in the "Drugs and the Pharmaceutical Sciences" series, Marcel Dekker. In one preferred aspect, the compositions are in the form of tablets, capsules, granules, pellets, troches, lozenges, and the like. Suitable enteric formulations are described, e.g., in U.S. Patent No. 5,350,741.

The pharmaceutical carrier or diluent employed may be a conventional solid or liquid carrier. Examples of solid carriers are lactose, terra alba, sucrose, cyclodextrin, talc, gelatin, agar, pectin, acacia, magnesium stearate, stearic acid or lower alkyl ethers of cellulose. Examples of liquid carriers are syrup, peanut oil, olive oil, phospholipids, fatty acids, fatty acid amines, polyoxyethylene and water.

Similarly, the carrier or diluent may include any sustained release material known In the art, such as glyceryl monostearate or glyceryl distearate, alone or mixed with a wax.

If a solid carrier is used for oral administration, the preparation may be tabletted, placed in a hard gelatin capsule in powder or pellet form, or it can be in the form of a troche or lozenge. The amount of solid carrier will vary widely but will usually be from about 25 mg to about 1 g.

If a liquid carrier is used, the preparation may be in the form of a syrup or liquid suitable for oral ingestion.

It will be appreciated that the actual preferred amounts of active compounds used in a given therapy will vary according to e.g. the specific compound being utilized, the particular composition formulated, the mode of administration and characteristics of the subject, e.g. the species, sex, weight, general health and age of the subject, Optimal administration rates for a given protocol of administration can be readily ascertained by those skilled in the art using conventional dosage determination tests conducted with regard to the foregoing guidelines. Suitable dose ranges may include from about 1 mg/kg to about 100mg/kg of body weight per day.

Therapeutic compounds of the invention are suitably administered in a protonated and water-soluble form, e.g., as a pharmaceutically acceptable salt, typically an acid addition salt such as an inorganic acid addition salt, e.g.,

a hydrochloride, sulfate, or phosphate salt, or as an organic acid addition salt such as an acetate, maleate, fumarate, tartrate, or citrate salt. Pharmaceutically acceptable salts of therapeutic compounds of the invention also can include metal salts, particularly alkali metal salts such as a sodium salt or potassium salt; alkaline earth metal salts such as a magnesium or calcium salt; ammonium salts such an ammonium or tetramethyl ammonium salt; or an amino acid addition salts such as a lysine, glycine, or phenylalanine salt.

The compounds of the invention may also be administered topically to treat peripheral vascular diseases and as such may be formulated as a cream or ointment.

The present peptides may also be formulated in compositions such as sterile solutions or suspensions for non-oral administration. Peptides of the invention may be administered parenterally, that is by intravenous, intramuscular, subcutaneous, intranasal, intrarectal, intravaginal or intrapritoneal administration.

The present invention will now be further described by the following working examples, which illustrate preferred embodiments of the invention.

**Treatment Methods**

In one aspect, the invention provides a method of administering to a patient having, or at risk of developing, a condition associated with impaired GJIC, a therapeutically effective amount of any of the peptides described above. Preferably, administration is oral. Patients who may be treated using peptides according to the invention include, but are not limited to, animals, preferably mammals, e.g., rodents (including mice, rats, hamsters, and lagomorphs, such as rabbits), dogs, pigs, goats (generally any domestic animal), and primates. In one preferred aspect, a patient is a human being.

In another aspect the invention concerns the use of a peptide according to the invention for the manufacture of a medicament for the treatment of a pathological condition involving impaired gap junctional communication comprising administering to a patient a therapeutically effective amount of said peptide.

Examples of conditions which can be treated include, but are not limited to, cardiovascular disease, inflammation of airway epithelium, disorders of alveolar tissue, bladder incontinence, impaired hearing due to diseases of the cochlea, endothelial lesions, diabetic retinopathy and diabetic neuropathy, ischemia of the central nervous system and spinal cord, dental tissue disorders including periodontal disease, kidney diseases, failures of bone marrow transplantation, wounds, erectile dysfunction, urinary bladder incontinence, neuropathic pain, subchronic and chronic inflammation, cancer and failures of bone marrow and stem cell transplantation, conditions which arise during transplantation of cells and tissues or during medical procedures such as surgery; as well as conditions caused by an excess of reactive oxygen species and/or free radicals and/or nitric oxide.

<u>Arrhythmia</u>

In one preferred aspect, the invention provides a pharmacologically active antiarrhythmic peptide, and the use thereof, for treatment of arrhythmias and thrombotic complications arising during cardiovascular disorders, such as acute ischemic heart disease (e.g., stable angina pectoris, unstable angina pectoris, acute myocardial infarction), congestive heart failure (e.g., systolic, diastolic, high-output, low-output, right or left sided heart fallure), congenital heart diseases, cor pulmonale, cardiomyopathies, myocarditis, hypertensive heart disease, during coronary revascularization, and the like.

In specific embodiments, an antiarrhythmic peptide according to the present invention is used to treat and/or prevent bradyarrhythmias (e.g., due to disease in sinus node, AV node, bundle of His, right or left bundle branch), and tachyarrhythmias associated with reentry (e.g., atrial premature complexes, AV junctional complexes, ventricular premature complexes, atrial fibrillation, atrial flutter, paroxymal supraventricular tachycardia, sinus node reentrant tachycardia, AV nodal reentrant tachycardia, and non-sustained ventricular tachycardia) either alone or in combination with other antiarrhythmic compounds, such as class I agents (e.g., lidocaine), class II agents (e.g., metoprolol or propranolol), class III agents (e.g., amiodarone or sotalol) or class IV agents (e.g., verapamil).

Additionally, or alternatively, peptides according to the invention are used to treat one or more of: a reentry arrhythmia; ventricular reentry (e.g., such as arises during acute myocardial infarction, chronic myocardial infarction, stable angina pectoris and unstable angina pectoris); infectious or autonomic cardiomyopathy; atrial fibrillation;repolarization alternans; monomorphic ventricular tachycardia; T-wave alternans; bradyarrhythmias; and generally, reduced contractility of cardiac tissue, thrombosis and the like.

<u>Osteoporosis</u>

In a further aspect, peptides according to the invention are used to prevent and/or treat osteoporosis or other pathologies affecting bone formation, growth or maintenance. Peptides which are able to normalize the attenuated GJIC

between human osteoblast during hypoxia are particularly suitable for the treatment of bone diseases with impaired bone formation relative to bone resorption. Optimal peptides for use in such methods can be selected in assays for increased alkaline phosphatase (ALP) activity in osteoblasts, which provides a means to monitor cell viability and growth as a consequence of proper maintenance of GJIC. In one aspect, human osteoblasts are stimulated with different concentrations of peptides from $1 \times 10^{-13}$ to $1 \times 10^{-6}$, and compared to untreated controls. Under normal culture conditions, peptides preferably increase ALP activity. Even more preferably, the peptides stimulate ALP activity during hypoxic conditions at concentrations ranging from $10^{-11}$ to $10^{-8}$ mol/l. The assay can thus be used to optimize peptide compositions for the treatment and/or prevention of bone diseases associated with poor vascularization, hypoxia and ischemia in bone tissue.

In another aspect, peptides according to the invention are used for the prevention and/or treatment of joint diseases that involves impaired cell-to-cell coupling. For example, the peptides can be used for the prevention and/or treatment of joint diseases that involve metabolic stress. These would include any form of arthritis associated with decreased vascularization or healing of fractured cartilage tissue.

Cancer

In still another aspect, peptides according to the invention are used to treat cancer. Carcinogenesis is characterized by the progressive impairment of growth control mechanisms in which growth factors, oncogenes and tumor suppressor genes are involved. A general theme in carcinogenesis and tumorigenesis is the down regulation of the GJIC. Permeability of gap junctions in tumor cells using the dye-transfer assay is typically lower than GJIC in surrounding tissue. Further, the gating of gap junctions is known to be effected by tumor promoters, which decrease GJIC. Therefore, in one aspect, peptides according to the invention are used as medicaments for the treatment of cancer, alone, or in conjunction with traditional anti-cancer therapies.

Wounds

In a further aspect, peptides according to the invention are used to treat wounds and, in particular, to accelerate wound healing. Wound healing involves the interactions of many cell types, and intercellular communication mediated by gap junctions is considered to play an important role in the coordination of cellular metabolism during the growth and development of cells involved in tissue repair and regeneration (K. M. Abdullah, et al. (1999) Endocrine, 10 35-41; M. Saitoh, et al. (1997) Carcinogenesis, 18: 1319-1328; J. A. Goliger, et al. (1995) Mol.Biol.Cell, 6 1491-1501). Peptides may be administered to the site of a wound by topical administration using carriers well known in the art (e.g., ointments, creams, etc.) or may administered systemically, e.g., for treating wounds of internal tissues, such as in the treatment of chronic gastric ulcer lesions.

Additional functions in which endothelial gap-junctional intercellular communication has been implicated are the migratory behavior of endothelial cells after injury, angiogenesis, endothelial growth and senescence, and the coordination of vasomotor responses (G. J. Christ, et al. (2000) Braz. J Med Biol.Res., 33: 423-429). Therefore, in one aspect, a peptide according to the invention is used to enhance conducted vascular responses and to improve blood supply during conditions with increased metabolic demand (e.g., physical exercise, tachycardia), and during ischemia.

Gap junctions are also believed to provide the molecular link for co-ordinated long-range signaling among individual members of the glial compartment. Likewise, astrocytes are ideally suited for metabolic support of neurons since they are functionally polarized with one extremity touching the vascular bed and the other pole approximates neuronal parenchyma (R. Dermietzel (1998) Brain Res. Brain Res. Rev., 26: 176-183). Therefore, in one preferred embodiment, peptides according to the invention are administered to a patient in need to prevent ischemic damage in the brain by increasing the metabolic support between glia cells and neurons. Such patients may include patients with organic psychoses, which may present with signs such as depression, anxiety, learning and memory deficit, phobias, and hallucinations or patients who have suffered a traumatic brain injury. Preferably, such peptides are selected or formulated so as to be available to the central nervous system (i.e., provided with or conjugated with carriers which facilitate transport across the blood-brain barrier).

Peptides according to the invention may also be used to accelerate repair after nerve injury or during grafting of immature cells (progenitor cells) into brain tissue, e.g., such as in patients with neurotrauma, brain ischemia and chronic neurodegenerative diseases, such as Parkinson's disease or Huntington's disease (H. Aldskogius, et al. (1998) Prog. Neurobiol, 55: 1-26).

In specific embodiments, a peptide according to the present invention may, due to the effect on the intercellular gap junction channels, be used to treat and/or prevent cataract (D. Mackay, et al. (1999) Am J Hum.Genet, 64 1357-1364) treat and/or prevent vascularization of the cornea in disease states with poor nutrition of the cornea and increase the healing of corneal lesions (S. G. Spanakis, et al. (1998) Invest Ophthalmol.Vis.Sci., 39: 1320-1328) and/or prevent hypertension.

It should be obvious to those of skill in the art, that the peptides and pharmaceutical compositions according to the invention can be used to treat any condition or pathology associated with impaired (abnormal decreases or increases in) gap junctional communication. Preferably, one or more of the peptides or pharmaceutical compositions comprising the one or more peptides are administered to a patient in need thereof in a therapeutically effective amount. As used herein, "a therapeutically effective amount" is one which reduces symptoms of a given condition or pathology, and preferably which normalizes physiological responses in a patient with the condition or pathology. Reduction of symptoms or normalization of physiological responses can be determined using methods routine in the art and may vary with a given condition or pathology. In one aspect, a therapeutically effective amount of one or more peptides or pharmaceutical composition comprising the one or more peptides is an amount which restores a measurable physiological parameter to substantially the same value (preferably to within $\pm$ 30%, more preferably to within $\pm$ 20%, and still more preferably, to within 10% of the value) of the parameter in a patient without the DMF.

## EXAMPLES

The invention will now be further illustrated with reference to the following examples. It will be appreciated that what follows is by way of example only and that modifications to detail may be made while still falling within the scope of the invention.

### Example 1. Peptide Synthesis

A preferred general procedure is described below. However, more detailed descriptions of solid phase peptide syntheses are found in WO98/11125 hereby incorporated in its entirety.

#### a. General Peptide Synthesis

*Apparatus and synthetic strategy*

Peptides were synthesized batchwise in a polyethylene vessel equipped with a polypropylene filter for filtration using 9-fluorenylmethyloxycarbonyl (Fmoc) as N-$\alpha$-amino protecting group and suitable common protection groups for side-chain functionalities.

*Solvents*

Solvent DMF (*N,N*-dimethylformamide, Riedel de-Häen, Germany) was purified by passing through a column packed with a strong cation exchange resin (Lewatit S 100 MB/H strong acid, Bayer AG Leverkusen, Germany) and analyzed for free amines prior to use by addition of 3,4-dihydro-3-hydroxy-4-oxo-1,2,3-benzotriazine (Dhbt-OH) giving rise to a yellow color (Dhbt-$O^-$ anion) if free amines are present. Solvent DCM (dichloromethane, analytical grade, Riedel de-Häen, Germany) was used directly without purification. Acetonitril ( HPLC-grade, Lab-Scan, Dublin Ireland) was used directly without purification.

*Amino Acids*

Fmoc-protected amino acids were purchased from Advanced ChemTech (ACT) in suitabel side-chain protected forms. Otherwise protected amino acids (Boc-Asp(OFm)-OH, Boc-D-Asp(OFm)-OH, Fmoc-Lys(Aloc)-OH, Fmoc-D-Lys (Aloc)-OH, Boc-Lys(Fmoc)-OH Boc-D-Lys(Fmoc)-OH, Boc-Orn(Fmoc)-OH from Bachem (Switzerland); Fmoc-Lys (ivDde)-OH, Fmoc-Sar-OH from NovaBiochem (Switzerland).

*Benzoic acid and Benzyl Amine Derivatives*

Benzoic acid and Benzyl amine derivatives were purchased from Aldrich and used without further purification.

*Coupling Reagents*

Coupling reagent diisopropylcarbodiimide (DIC) was purchased from (Riedel de-Häen, Germany), PyBop from Advanced ChemTech.

*Linkers*

(4-hydroxymethylphenoxy)acetic acid (HMPA), was purchased from Novabiochem, Switzerland; and was coupled to the resin as a preformed 1-hydroxybenzotriazole (HOBt) ester generated by means of DIC.

*Solid Supports*

Peptides synthesized according to the Fmoc-strategy on TentaGel S resins 0,22-0,31 mmol/g (TentaGel-S-$NH_2$; TentaGel S-Ram, Rapp polymere, Germany).

*Catalysts and Other Reagents*

Diisopropylethylamine (DIEA) was purchased from Aldrich, Germany, and ethylenediamine from Fluka, piperidine and pyridine from Riedel-de Häen, Frankfurt, Germany. 4-(N,N-dimethylamino)pyridine (DMAP) was purchased from Fluka, Switzerland and used as a catalyst in coupling reactions involving symmetrical anhydrides. Ethandithiol was purchased from Riedel-de Häen, Frankfurt, Germany. 3,4-dihydro-3-hydroxy-4-oxo-1,2,3-benzotriazine (Dhbt-OH), 1-hydroxybenzotriazole (HOBt) (HOAt) were obtained from Fluka, Switzerland.

*Coupling Procedures*

The first amino acid can be coupled as a symmetrical anhydride in DMF generated from the appropriate n-α-protected amino acid and the subsequent amino acids can be coupled as *in situ* generated HOBt or HOAt esters made from appropriate n-α-protected amino acids and HOBt or HOAt by means of DIC in DMF. The acylations were checked by the ninhydrin test performed at 80 oc in order to prevent Fmoc deprotection during the test (B. D. Larsen, A. Holm, Int.J Pept.Protein Res. 1994, 43 1-9).

*Deprotection of the N-α-amino Protecting Group (Fmoc)*

Deprotection of the Fmoc group was performed by treatment with 20% piperidine in DMF (1x5 and 1x10 min.), followed by wash with DMF (5 x 15 ml, 5 min. each) until no yellow color could be detected after addition of Dhbt-OH to the drained DMF.

*Deprotection of Allyl/Aloc*

A solution of 3 eq. $Pd(PPh_3)_4$ dissolved in 15-20 ml $CHCl_3$, AcOH, NMM (37:2:1) was added to the peptid resin. The treatment was continued for three hours at room temperature accompanied by bubbling a stream of $N_2$ through the mixture.

*Coupling Of Hobt-Esters*

3 eq. N-α-amino protected amino acid was dissolved in DMF together with 3 eq. HOBt and 3 eq. DIC and then added to the resin.

*Preformed Symmetrical Anhydride*

Six eq. N-α-amino protected amino acid was dissolved in DCM and cooled to 0˚C. DIC (3 eq.) was added and the reaction continued for 10 minutes. The solvent was removed in vacuo and the remanence dissolved in DMF. The solution was immediately added to the resin followed by 0.1 eq. of DMAP.

*Cleavage Of Peptide From Resin With Acid*

Peptides were cleaved from the resins by treatment with 95% triflouroacetic acid (TFA, Riedel-de Häen, Frankfurt, Germany)-water v/v or with 95% TFA and 5% ethandithiol v/v at r.t. for 2 hours. The filtered resins were washed with 95% TFA-water and filtrates and washings evaporated under reduced pressure. The residue was washed with ether and freeze-dried from acetic acid-water. The crude freeze-dried product was analyzed by high-performance liquid chromatography (HPLC) and identified by electrospray ionisation mass spectrometry (ESMS).

*Batchwise Peptide Synthesis on TentaGel Resin (PEG-PS)*

TentaGel resin (1g, 0.22-0.31 mmol/g) was placed in a polyethylene vessel equipped with a polypropylene filter for filtration. The resin was swelled in DMF (15ml), and treated with 20% piperidine in DMF to secure the presence of non-protonated amino groups on the resin. The resin was drained and washed with DMF until no yellow color could be detected after addition of Dhbt-OH to the drained DMF. HMPA (3 eq.) was coupled as a preformed HOBt-ester as described above and the coupling was continued for 24 h. The resin was drained and washed with DMF (5 x 5 ml, 5 min each) and the acylation checked by the ninhydrin test. The first amino acid was coupled as a preformed symmetrical anhydride as described above. The following amino acids according to the sequence were coupled as preformed Fmoc-protected HOBt esters (3 eq.) as described above. The couplings were continued for 2 h, unless otherwise specified. The resin was drained and washed with DMF (5 x 15 ml, 5 min each) in order to remove excess reagent. All acylations were checked by the ninhydrin test performed at 80 ˚C. After completed synthesis the peptide-resin was washed with DMF (3x15 ml, 5 min each), DCM (3x15 ml, 1 min each) and finally diethyl ether (3x15 ml, 1 min each) and dried *in vacuo.*

*Preparative HPLC conditions.*

Preparative chromatography was carried out using a VISION Workstation (PerSeptive Biosystem) equipped with AFC2000 automatic fraction collector/autosampler. VISION-3 software was used for instrument control and data acquisition.

*Column*

Kromasil (EKA Chemicals) KR100-10-C8 100A, C-8, 10 □; CER 2230, 250 x 50,8 mm or a VYDAC 218TP101550, 300A, C-18, 10-15 □, 250 x 50 mm. The buffer system used included A: 0,1% TFA in MQV; B: 0,085% TFA, 10% MQV, 90% MeCN. Flow rates were 35-40 ml/min and the column temperature was 25˚C. UV detection was performed at 215 nm and 280 nm. Suitable gradients were optimized for individual peptides.

*Analytical HPLC Conditions*

Gradient HPLC analysis was done using a Hewlett Packard HP 1100 HPLC system consisting of a HP 1100 Quaternary Pump, a HP 1100 Autosampler a HP 1100 Column Thermostat and HP 1100 Multiple Wavelength Detector. Hewlett Packard Chemstation for LC software (rev. A.06.01) was used for instrument control and data acquisition.

For analytical HPLC, different columns were used as appropriate, such as VYDAC 238TP5415, C-18, 5µm, 300A, or a Jupiter, Phenomenex 00F-4053-E0; 5 □m C-18, 300A 150 x 4,6 mm and others. The buffer system included A: 0,1% TFA in MQV; B: 0,085% TFA, 10% MQV, 90% MeCN. Flow rates were 1 ml/min. The preferred column temperature was 40˚C. UV detection was performed at 215 nm. As above, suitable gradients were optimized for the individual peptides.

*Mass Spectroscopy*

The peptides were dissolved in super gradient methanol (Labscan, Dublin, Ireland), Milli-Q water (Millipore, Bedford, MA) and formic acid (Merck, Damstadt, Germany) (50:50:0.1 v/v/v) to give concentrations between 1 and 10 µg/m). The peptide solutions (20 µl) were analysed in positive polarity mode by ESI-TOF-MS using a LCT mass spectrometer (Micromass, Manchester, UK) accuracy of +/- 0.1 m/z.

*General synthetic procedure*

In all syntheses, dry TentaGel-S-$NH_2$ (0.23 mmol/g, 1g) was placed in a polyethylene vessel equipped with a polypropylene filter for filtration and treated as described under "batchwise peptide synthesis on TentaGel resin". Lysine and analogs thereof (e.g. Ornithin, 2,4-Diaminobutanoic acid, 1,3-diaminopropanoic acid etc.) when situated C-terminally were coupled as the N-α, Fmoc protected derivatives with either ivDde or Aloc protection of the side chain functionality (e.g. Fmoc-Lys(Aloc)-OH). Lysine and analogs thereof when situated N-terminally were coupled as the N-α Boc protected derivatives with Fmoc protection of the side chain functionality (e.g. Boc-Lys(Fmoc)-OH). Aspartic acid, Glutamic acid and analogs thereof when situated C-terminally were coupled as N-α Fmoc protected derivatives with Allylic protection of the side chain functionality (e.g. Fmoc-Asp(Oallyl)-OH). Aspartic acid, Glutamic acid and analogs thereof when situated N-terminally were coupled as N-α Boc protected derivatives with Fm protection of the side chain functionality (e.g. Boc-Asp(OFm)-OH). Other amino acids than the above mentioned when situated C-terminally were coupled as N-α Fmoc protected derivatives with suitable protection of the side chain functionalities or when situated N-terminally as N-α Boc protected derivatives with suitable protection of the side chain functionalities.

In all cases the dipeptide was assembled followed by deprotection of the side chain protecting group of the

Lysine, Aspartic- or Glutamic acid or analogs thereof.

In case of Lysine or analogs thereof, the hydrophobic group functionalised as a carboxylic acid was coupled as an *in situ* generated HOBt ester by means of DIC in THF.

In case of Aspartic- and Glutamic acid or analogs thereof, the hydrophobic group functionalised as an amine was coupled to the pre generated HOBt ester of the side chain carboxylic acid by means of DIC in DMF catalyzed by triethylamine.

All couplings were continued for at least 2 hours. The acylations were checked by the ninhydrin test performed at 80 ˚C as earlier described. After completed synthesis the peptide-resin was washed with DMF (3x 15 ml, 1 min each), DCM (3x 15 ml, 1 min each), diethyl ether (3x 15 ml, 1 min each) and dried *in vacuo.* The peptide was then cleaved from the resin as described above and freeze-dried.

After purification using preparative HPLC as described above, the peptide product was collected and the identity of the peptide was confirmed by ES-MS .

The above procedure was used for the synthesis of all peptides exemplified further below and the peptides shown in Table 1 of the specification. Exemplary synthesis schemes are shown in Figures 1A and 1B.

**b. synthesis of Individual Peptides**

*Synthesis of H-Gly-Lys (4-nitrobenzoyl)-OH (Compound 1)*

Lysine was coupled as Fmoc-Lys(ivDde)-OH and the N-terminal Glycine as the Boc derivative. The ivDde protecting group was removed as described above. The Lysine and Glycine were first assembled on the solid support. The ivDdde group was then removed and subsequently 4-nitrobenzoic acid was coupled as an *in situ* generated HOBt ester by means of DIC in THF. After purification using preparative HPLC as described above, 35 mg peptide product was collected with a purity greater than 99 %. The identity of the peptide was confirmed by ES-MS (found $MH^+$ 352.28, calculated $MH^+$ 352.21).

*Synthesis of H-Gly-Lys(4-cyanobenzoyl)-OH (Compound 3)*

Lysine was coupled as Fmoc-Lys(ivDde)-OH and the N-terminal Glycine as the Boc derivative. The ivDde protecting group was removed as described above. The Lysine and the Glycine were first assembled on the solid support. The ivDdde group was then removed and subsequently 4-cyanobenzoic acid was coupled as an *in situ* generated HOBt ester by means of DIC in DMF. After purification using preparative HPLC as described above, 6 mg peptide product was collected with a purity greater than 90 %. The identity of the peptide was confirmed by ES-MS (found $MH^+$ 332.25, calculated $MH^+$ 332.21).

*Synthesis of H-Gly-Lys(4-methoxybenzoyl)-OH (Compound 4)*

Lysine was coupled as Fmoc-Lys(ivDde)-OH and the N-terminal Glycine as the Boc derivative. The ivDde protecting group was removed as described above. The Lysine and the Glycine were first assembled on the solid support. The ivDdde group was then removed and subsequently 4-methoxybenzoic acid was coupled as an *in situ* generated HOBt ester by means of DIC in DMF. After purification using preparative HPLC as described above, 7 mg peptide product was collected with a purity greater than 95 %. The identity of the peptide was confirmed by ES-MS (found $MH^+$ 337.28, calculated $MH^+$ 337.21).

*Synthesis of H-Lys(4-nitrobenzoyl)-Gly-OH (Compound 7)*

Glycine was coupled as Fmoc-Glycine and the N-terminal Lysine was coupled as Boc-Lys(Fmoc)-OH. The Glycine and the Lysine were first assembled on the solid support. The

Fmoc in the side chain of Lysine was then removed and subsequently 4-nitrobenzoic acid was coupled as an *in situ* generated HOBt ester by means of DIC in THF. After purification using preparative HPLC as described above, 64 mg peptide product was collected with a purity greater than 98 %. The identity of the peptide was confirmed by ES-MS (found $MH^+$ 352.19, calculated $MH^+$ 352.24).

*Synthesis of H-D-Lys(4-methoxybenzoyl)-Gly-OH (Compound 21)*

Glycine was coupled as Fmoc-Glycine and the N-terminal Lysine was coupled as Boc-D-Lys(Fmoc)-OH. The Glycine and the Lysine were first assembled on the solid support. The Fmoc in the side chain of Lysine was then removed and subsequently 4-methoxybenzoic acid was coupled as an *in situ* generated HOBt ester by means of DIC in DMF.

After purification using preparative HPLC as described above, 31 mg peptide product was collected with a purity greater than 97 %. The identity of the peptide was confirmed by ES-MS (found $MH^+$ 337.24, calculated $MH^+$ 337.21).

*Synthesis of H-D-Lys(4-nitrobenzoyl)-Gly-OH (Compound 22)*

Glycine was coupled as Fmoc-Glycine and the N-terminal Lysine was coupled as Boc-D-Lys(Fmoc)-OH. The Glycine and the Lysine were first assembled on the solid support. The Fmoc in the side chain of Lysine was then removed and subsequently 4-nitrobenzoic acid was coupled as an *in situ* generated HOBt ester by means of DIC in THF. After purification using preparative HPLC as described above, 65 mg peptide product was collected with a purity greater than 98 %. The identity of the peptide was confirmed by ES-MS (found MH+ 352.27, calculated MH+ 352.24).

*Synthesis of H-D-Lys(benzoyl)-Gly-OH (Compound 23)*

Glycine was coupled as Fmoc-Glycine and the N-terminal Lysine was coupled as Boc-D-Lys(Fmoc)-OH. The Glycine and the Lysine were first assembled on the solid support. The Fmoc in the side chain of Lysine was then removed and subsequently Benzoic acid was coupled as an *in situ* generated HOBt ester by means of DIC in DMF. After purification using preparative HPLC as described above, 31 mg peptide product was collected with a purity greater than 96 %. The identity of the peptide was confirmed by ES-MS (found MH+ 307.22, calculated MH+ 307.24).

*Synthesis of H-D-Lys(4-t-Butylbenzoyl)-Gly-OH (Compound 54)*

Glycine was coupled as Fmoc-Glycine and the N-terminal Lysine was coupled as Boc-D-Lys(Fmoc)-OH. The Glycine and the Lysine were first assembled on the solid support. The Fmoc in the side chain of Lysine was then removed and subsequently 4-t-Butylbenzoic acid was coupled as an *in situ* generated HOBt ester by means of DIC in DMF. After purification using preparative HPLC as described above, 34 mg peptide product was collected with a purity greater than 97 %. The identity of the peptide was confirmed by ES-MS (found MH+ 363.27, calculated MH+ 363.22).

*Synthesis of H-Asn(NH(4-methoxybenzyl))- D-Alanine-OH (Compound 80)*

Alanine was coupled as Fmoc-D-Alanine and the N-terminal Asparagine was coupled as Boc-Asp(OFm)-OH. The Alanine and the Aspartic acid were first assembled on the solid support. The Fm in the side chain of Aspartic acid was then removed and subsequently 4-methoxybenzylamine was coupled catalyzed by triethylamine to the pre generated HOBt ester of the side chain carboxylic acid by means of DIC in DMF. After purification using preparative HPLC as described above, 17 mg peptide product was collected with a purity greater than 98 %. The identity of the peptide was confirmed by ES-MS (found MH+ 323.17, calculated MH+ 323.15).

*Synthesis of H-D-Asn(NH(4-methoxybenzyl))-Alanine-OH (Compound 95)*

Alanine was coupled as Fmoc-Alanine and the N-terminal Asparagine was coupled as Boc-D-Asp(OFm)-OH. The Alanine and the Aspartic acid were first assembled on the solid support. The Fm in the side chain of Aspartic acid was then removed and subsequently 4-methoxybenzylamine was coupled catalyzed by triethylamine to the pre generated HOBt ester of the side chain carboxylic acid by means of DIC in DMF. After purification using preparative HPLC as described above, 25 mg peptide product was collected with a purity greater than 98 %. The identity of the peptide was confirmed by ES-MS (found MH+ 323.12, calculated MH+ 323.15).

*Synthesis of H-D-Asn(NH(4-nitrobenzyl))-Alanine-OH (Compound 96)*

Alanine was coupled as Fmoc-Alanine and the N-terminal Asparagine was coupled as Boc-D-Asp(OFm)-OH. The Alanine and the Aspartic acid were first assembled on the solid support. The Fm in the side chain of Aspartic acid was then removed and subsequently 4-nitrobenzylamine was coupled catalyzed by triethylamine to the pre generated HOBt ester of the side chain carboxylic acid by means of DIC in DMF. After purification using preparative HPLC as described above, 102 mg peptide product was collected with a purity greater than 99 %. The identity of the peptide was confirmed by ES-MS (found MH+ 338.14, calculated MH+ 338.12).

**Example 2 Bioavailability**

MATERIALS AND METHODS

Pharmacokinetic screening and food interaction studies

*Chemicals & Materials*

The water used for these experiments was of highest quality obtained from a reversed osmosis primary system in combination with a Milli-Q water secondary treatment system (Millipore, Bedford, MA, USA). Methanol was super gradient quality obtained from Labscan Ltd. (Dublin, Ireland). Formic acid p.a. (98-100%) was obtained from Merck (Darmstadt, Germany). Heptafluorobuturicacid, HPLC grade wAs obtained from Pierce (Rockford, III, USA). EDTA stabilised plasma from rat (Sprague-Dawley) was obtained from Harlan Sera Lab Ltd. (Loughborough, UK). Blood samples were collected in potassium EDTA coated microtainers from BD Vacutainer Systems (Plymouth, UK). Sample preparation by ultra filtration was performed using Microcon centrifugal filter devices with a molecular weight cut off of 3000 obtained from Millipore (Bedford, MA, USA).

*Instrumentation*

The LC/MS/MS analysis was performed on a Waters Alliance 2790 HPLC instrument in combination with a Quattro Ultima mass spectrometer from Micromass (Manchester, UK). Both the LC and MS were controlled by MassLynx 3.5 software. The LC separations prior to MS/MS detection were performed on an XTerra MS $C_{18}$ (2.0 x 50 mm), 3.5 μm particles, (Waters, Milford, MA, USA).

*Animals*

Twelve male Sprague-Dawley rats (approx 350 g) were obtained from M&B (Denmark) and catheters were inserted into the femoral vein and artery during Hypnorm®-Dormicum® anaesthesia. After surgery, the rats were allowed to recover for five days before drug administration was initiated.

*Drugs and Dose Levels*

The dipeptides (Compound 22, Compound 21, Compound 23, Compound 96, Compound 95 and Compound 54) were obtained as the TFA salts and dissolved in PBS (2.6 mM KCl, 137 mM NaCl, 1.5 mM $KH_2PO_4$ and 8.2 mM $Na_2HPO_4$ adjusted to pH=7.4) to give concentrations of 500 μM. The dosing volumes were 1 mL/kg corresponding to doses of 500 nmol/kg, respectively. After each experiment the dosing solutions were diluted in 0.5% formic acid in water and analysed by LC/MS/MS for their content of drug substance. The concentrations of drug substance in the dosing solutions were calculated from the responses of standards in the range from 1 to 1000 nM.

*Study Design*

The drug substances were administered to two animals as i.v. and p.o. bolus in a cross-over study design. The p.o. dose was administered to rats fasted for a 17 hours period prior to drug administration. The animals were allowed to rest for 48 hours in between drug administrations. After the first experiment the rats received a blood transfusion with heparin treated blood.

Prior to drug administration (5 min) the rats received 500 IU heparin as an i.v. bolus and a control blood sample was collected. After drug administration blood samples of approx. 250 μL were collected from the femoral artery at the following time points; before dose (B.D.), 5, 15, 30, 60, 120, 180 and 240 min after i.v. administration and at B.D., 10, 30, 50, 80, 120, 180, 240, and 300 min after p.o. administration. The blood samples were stored on ice until centrifugation for 5 min at 10.000 x g (4˚C) and the plasma (100 μL) were transferred to 1.5 mL polypropylene tubes and stored at -20˚C until sample preparation and LC/MS/MS analysis.

*Sample Preparation and LC/MS/MS Analysis*

The plasma samples were thawed on ice, and 100 μL plasma was mixed with 100 μL 1 % (v/v) formic acid and transferred to microcon YM-3 filter units. The samples were then centrifuged for 1 hr at 8.300 x g at room temperature. The filtrates were collected in 300 μL autosampler vials and stored at 4˚C until injection (40 μL) onto the LC column. The chromatography was performed at 50˚C using a linear gradient from 100 % buffer A (0.1% formic acid in water) to

100% buffer B (0.1% formic acid in acetonitrile) in 4 min, followed by 3 min wash using buffer B and finally a reequilibration period for 7 min using buffer A. The detection of the drug substances were performed by MS/MS using argon as collision gas (1.3 x $10^{-3}$ mbar). The specific MS/MS settings for each of the tested compounds are given in Table 2.

**Table 2:** MS/MS settings used for the detection of compounds x-y in the bioavailability study.

| Compound | Cone voltage | Collision Energy | Parent ion | Daughter ion(s) |
|---|---|---|---|---|
| 22 | 40 V | 24 eV | 353.15 m/z | 150.00 m/z |
| 21 | 60 V | 28 eV | 338.15 m/z | 135.00 m/z |
| 23 | 30 V | 18 eV | 308.10 m/z | 187.9 m/z<br>104.9 m/z |
| 96 | 45 V | 15 eV | 339.15 m/z | 220.00 m/z<br>169.80 m/z<br>123.90 m/z |
| 95 | 60 V | 20 eV | 324.10 m/z | 120.90 m/z |
| 54 | 60 V | 26 eV | 364.20 m/z | 160.90 m/z |

*Data Analysis*

The plasma concentrations of the drug substance were calculated from the area related to an external calibration curve obtained from spiked plasma samples treated as described above. The calibration curve was obtained by linear regression of the log(peak area) vs. log(concentration). The calibration curve covered the concentration range from 0.1 to 100 nM.

The plasma concentrations versus time data from each animal was used for pharmacokinetic modelling in WinNonLin 3.5 (Pharsight, Mountain View, CA, USA) using non-compartmental analysis and the value of the area under the plasma concentrartion curve (AUC) were reported. The oral bioavailability $F_{po}$ was calculated as the ratio in % between the dose (D) normalised AUC's after iv and po administration: $AUC_{po}*D_{iv}/AUC_{iv}*D_{po.}$

RESULTS

The oral bioavailability of 6 compounds was tested and all compounds were detected after oral administration. The calculated bioavailabilities are summarised in Table 3. The bioavailability of Compounds 22, 23, 96, and 95 were moderate (within 10-31%) and the bioavailability of Compounds 54 and 21 were low (within 2 - 8%).

**Table 3:** The mean bioavailability ± standard error on mean (SEM) of the 6 tested compounds.

| Compound No. | F (%) | |
|---|---|---|
| | Mean | SEM |
| 22 | 31 | 5.3 |
| 21 | 2.6 | 0.78 |
| 23 | 10 | 5.7 |
| 96 | 15 | 3.0 |
| 95 | 29 | 10 |
| 54 | 7.2 | 0.40 |

CONCLUSION

The peptides of the invention showed oral availability having a range of availability in Sprague-Dawley rats between 2 an 31%.

**Example 3 Osteoblast Activity (ALP assay)**

MATERIALS AND METHODS

Human primary osteoblasts were obtained from bone marrow biopsies of the posterior iliac spine from voluntary donors. Cells were used at 6 weeks of culturing. Cells were cultured in 24-well plates in an 8 days process and stimulated with the test compounds in concentrations from $1x10^{-13}$ to $1x10^{-6}$ for the last 4 days of the experiment. Cell lysate was collected for determination of ALP activity. hOB cells from 10-15 donors were used for the experiments. For ALP measurements a commercially available kit (Alkaline Phosphatase Kit, Sigma-Aldrich Denmark A/S) was used. All determinations were performed according to the manufacturer's recommendations.

Analyses

ALP was determined on cell lysate collected at termination of the experiment. Measurements were compared with and to vehicle treated control cultures.

*Data analysis*

Data are presented as means and standard deviations or medians and percentiles as appropriate. Two-way ANOVA is performed using Fisher's LSD test for post-hoc comparisons, using a 0.05 level of significance.

RESULTS

**Table 4**

| Compound | N | Mean | SD | 0.05 int |
|---|---|---|---|---|
| *22* | 4 | 1,12 | 0,05 | 0,05 |
| *1* | 4 | 1,08 | 0,07 | 0,06 |
| *23* | 5 | 1,08 | 0,07 | 0,06 |
| *95* | 5 | 1,14 | 0,09 | 0,08 |

The activity of Compounds 1, 22, 23, and 95 are illustrated in Figure 2 and summarized in the above Table 4.

CONCLUSION

The peptides of the invention increased the activity of human osteoblasts in primary culture as measured by an increase in production of alkaline phosphatase.

**Example 4 Standard Calcium-Induced Arrhythmia Assay**

The anti-arrhythmic effect of the dipeptides was determined in a model of calcium-induced arrhythmias according to the model of Lynch *et al.* [6]. Male NMRI mice (25-30 g; Bomholdtgaard, Ll. Skendsved, Denmark) were anaesthetised with a neurolept anaesthetic combination (Hypnorm® (fentanyl citrate 0.315 mg/ml and fuanisone 10 mg/ml) + midazolam (5 mg/ml)). Commercial solutions of Hypnorm® and midazolam were diluted 1:1 in distilled water, and one part diluted Hypnorm® was mixed with one part diluted midazolam. Anaesthesia was induced by s.c. administration of this solution in a dose of 50 - 75 μl/10 gram mouse.

An i.v. cannula was inserted into the tail vein. The lead II ECG signal was recorded continuously by positioning of stainless steel ECG electrodes on the right forelimb and on the left hind limb. The ground electrode was placed on the right hind limb. The signal was amplified (x 5.000-10.000) and filtered (0.1-150 Hz) via a Hugo Sachs Electronic model 689 ECG module. The analogue signal was digitised via a 12-bit data acquisition board (Data Translation model DT321) and sampled at 1000 Hz using the Notocord HEM 3.1 software for Windows NT. After a 10-min equilibration period, the test sample of drug was injected into the tail vein and three minutes later i.v. infusion of $CaCl_2$ (30 mg/ml, 0.1 ml/min ≈ 100 mg/kg/min), (calciumchlorid-2-hydrat, Riedel-de Haën, Germany) was started.

Mice pre-treated with vehicle (phosphate buffered saline with 0.1% bovine albumin) were tested on all days

as a measure for the control level in untreated animals. Injection volume was 100 μl in all experiments. The time lag to onset of arrhythmias ($t_{arr}$) was determined as the time from the start of $CaCl_2$ infusion until the first event of 2nd degree AV block (defined as intermittent failure of the AV conduction characterised by a P-wave without the concomitant QRS complex).

RESULTS

The % response of the tested compounds are given in Table 5. The given response is estimated according to ($t_{arr}$ (testcompound) - $t_{arr}$ (vehicle)) x 100/ $t_{arr}$ (vehicle).

**Table 5**

| Compound | % response | SEM |
|---|---|---|
| 1 | 52.0 | 8.0 |
| 3 | 37.0 | 9.0 |
| 4 | 69.0 | 11.0 |
| 7 | 40 | 7 |
| 21 | 55.0 | 4.0 |
| 22 | 52.0 | 12.0 |
| 23 | -8.0 | 7.0 |
| 54 | 61.3 | 12.3 |
| 80 | 33 | 6 |
| 95 | 0.3 | 9.4 |
| 96 | 54 | 6 |
| AAP | 18.9 | 6.4 |

CONCLUSION

The peptides of the invention showed both an antiarrhytmic effect and a non-antiarrhytmic effect.

## DEFINITIONS

Unless specified otherwise, the following definitions are provided for specific terms which are used in the following written description.

Throughout the description and claims the three-letter code for natural amino acids is used as well as generally accepted three letter codes for other α-amino acids, such as Sarcosin (Sar). Where the L or D form has not been specified, it is to be understood that the amino acid in question can be either the L or D form.

The term "halogen" refers to F, Cl, Br, and I, where F and I are preferred.

The term "alkyl" refers to univalent groups derived from alkanes by removal of a hydrogen atom from any carbon atom: $C_nH_{2n+1}$-. The groups derived by removal of a hydrogen atom from a terminal carbon atom of unbranched alkanes form a subclass of normal alkyl (n-alkyl) groups: $H[CH_2]_n$-. The groups $RCH_2$-, $R_2CH$- (R not equal to H), and $R_3C$- (R not equal to H) are primary, secondary and tertiary alkyl groups respectively. C(1-22)alkyl refers to any alkyl group having from 1 to 22 carbon atoms and includes C(1-6)alkyl, such as methyl, ethyl, propyl, iso-propyl, butyl, pentyl and hexyl and all possible isomers thereof.

By the phrase "lower alkyl" is meant a linear or branched alkyl having less than about 6 carbon atoms, preferably methyl, ethyl, propyl, or butyl.

The term "alkenyl" refers to a straight or branched or cyclic hydrocarbon group containing one or more carbon-carbon double bonds. C(2-22)alkenyl refers to any alkenyl group having from 1 to 22 carbon atoms and includes C(2-6) alkenyl, vinyl, allyl, 1-butenyl, etc.

The term "aralkyl" refers to aryl C(1-22)alkyl, and the term "aryl" throughout this specification means phenyl or naphthyl.

By the phrase "hydrophobic group" is meant an optionally substituted aromatic carbon ring, preferably a 6 or 12 -membered aromatic carbon ring. By the phrase "optionally substituted" is meant substitution of the 6 or 12-membered aromatic carbon ring with at least one of a lower alkyl, alkoxy, hydroxyl, carboxy, amine, thiol, hydrazide, amide, halide, hydroxyl, ether, amine, nitrile, imine, nitro, sulfide, sulfoxide, sulfone, thiol, aldehyde, keto, carboxy, ester, an amide group; including seleno and thio derivatives thereof. Also included in the definition of "optionally substituted" are are

sulfide, sulfoxide, sulfone, and thiol derivates with or without a seleno group. In embodiments in which the aromatic carbon ring is substituted such substitutions will typically number less than about 10 substitutions, more preferably about 1 to to 5 of same with about 1 or 2 substitutions being preferred for many invention applications. Preferred alkoxy groups include methoxy, ethoxy, and propoxy. Illustrative hydrophobic groups include unsubstituted benzyl, phenyl, and napthyl.

By the phrase "hydrogen bond group" is meant a donor or acceptor of a (non-covalent) hydrogen bond. In embodiments in which the hydrogen bond group is the donor, it will typically include at least one electronegative atom bound to a hydrogen atom. Examples of such electronegative atoms include, but are not limited to, nitrogen, oxygen, halide (eg., Cl, F, Br, etc), and sulfur. Illustrative hydrogen bond donors include hydroxyl, amine, thiol, hydrazide and amide. In embodiments in which the first hydrogen bond group is an acceptor, it will preferably include at least one electronegative atom such as those mentioned above in which the atom includes a non-bonded electron pair. Such pairs are often referred to as "lone pairs". Examples of preferred hydrogen bond acceptors include, but are not limited to, halide, hydroxyl, ether, amine, nitrile, imine, nitro, aldehyde, keto, carboxy, ester, amide group; and seleno derivatives thereof. Also envisioned are suitable sulfide, sulfoxide, sulfone, and thiol hydrogen bond acceptors including seleno derivatives thereof.

The terms "intercellular communication modulator", "gap junction facilitator", "compound that facilitates gap junction communication" and "gap junction opener", etc., all refer to a compound that facilitates, or maintains, or normalizes (e.g. either by inhibiting of enhancing), GJIC, irrespective of the particular mechanism behind this action. More specifically, the term "gap junction opener" may refer to a substance which normalizes (i.e., increases) the exchange of molecules that are able to pass through gap junctions between extracellular and intracellular spaces and/or which can normalize increase GJIC.

The term "agonist" refers to a peptide that can interact with a tissue, cell or cell fraction which is the target of for example, but not limited to, an AAP, AAP10, HP5 peptide, or functional analog thereof, to cause substantially, or at least substantially the same physiological responses in the tissue, cell or cell fractionas the AAP, AAP10, HP5 peptide, or functional analog thereof. In one aspect, the physiological response is one or more of: contraction, relaxation, secretion, and enzyme activation. Preferably, the peptide binds to the tissue, cell or cell fraction. In one aspect, the peptide binds to a receptor on the tissue, cell, or cell fraction, which binds to AAP, AAP10, HP5, or a functional analog thereof. However, the present "agonist" may interact with a tissue, cell or cell fraction, which is the target of any given peptide, causing the same, or at least substantially the same physiological response.

An "antiarrhythmic peptide agonist" as used herein is a peptide, which comprises an antiarrhythmic activity, which is substantially the same, or greater than, the antiarrhythmic activity of an AAP, AAP10, HP5 peptide or functional analog thereof. "Greater than" refers to an antiarrhythmic activity, which is observed at lower concentrations of peptide or in shorter periods of time compared to the antiarrhythmic activity of an AAP, AAP10, HP5 peptide or functional analog thereof.

The term "antagonist" refers to a peptide which inhibits or antagonizes one or more physiological responses observed in a tissue, cell or cell fraction after contacting the tissue, cell, or cell fraction with any given peptide, such as AAP, AAP10, HP5 peptide, or a functional analog thereof. In one aspect, the physiological response is one or more of: contraction, relaxation, secretion, and enzyme activation. Preferably, the peptide binds to the tissue, cell or cell fraction. In one aspect, the peptide binds to a receptor on the tissue, cell, or cell fraction which binds to AAP, AAP10, HP5, or a functional analog thereof and/or which inhibits binding of one or more of AAP, AAP10, HP5, a functional analog thereof to the receptor.

As used herein, "normalize" refers to a change in a physiological response such that the response becomes insignificantly different from one observed in a normal patient. Thus, normalization may involve an increase or decrease in the response depending on the pathology involved.

EC50 of less than about $10^{-6}$M, and preferably, less than about, $10^{-8}$ M.

As used herein "oral availability" refers to the rate and extent of absorption of an orally administered drug into the blood stream.

Abbreviations used in the instant application are defined further below.

| | | | |
|---|---|---|---|
| Methyl (Me) | $-CH_3$ | Methoxy (MaO) | $-OCH_3$ |
| Ethyl (Et) | $-CH_2CH_3$ | Ethoxy (ElO) | $-OCH_2CH_3$ |
| n-Butyl(n-Bu) | $-CH_2CH_2CH_2CH_3$ | n-Butoxy (n-BuO) | $-OCH_2CH_2CH_2CH_3$ |
| n-Hexyl (n-HeX) | $-CH_2CH_2CH_2CH_2CH_3$ | n-Hexyloxy (n-HexO) | $-OCH_2CH_2CH_2CH_2CH_2CH_3$ |
| n-Octyl (n-Oct) | $-CH_2CH_2CH_2CH_2CH_2CH_2CH_2CH_3$ | n-Octyloxy(n-OctO) | $-OCH_2CH_2CH_2CH_2CH_2CH_2CH_2CH_3$ |

(continued)

| | | | |
|---|---|---|---|
| t-Butyt (t-Bu) | 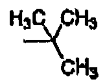 | t-Butoxy (t-BuO) | 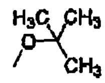 |
| cyclo-hexyl (c-Hex) | 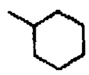 | cyclo-hexyloxy (c-HexO) | 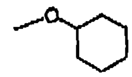 |
| Phenyl(Ph) | 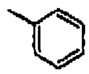 | Phenoxy(PhO) | 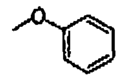 |
| Benzyl (Bzl) | 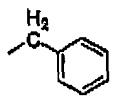 | Benzyloxy (BzlO) | 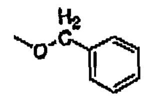 |

## Claims

**1.** A peptide for use in therapy represented by the general formula I:

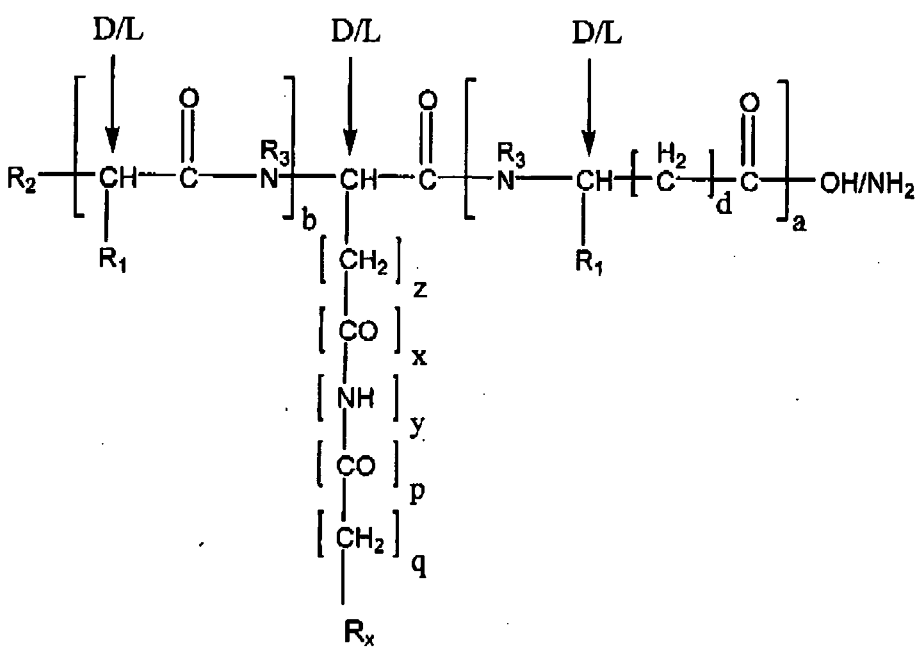

wherein:

a is 1 and b is 0; or
b is 1 and a is 0;
d is 0-8; and
z is 1-7; and
x is 1, y and q are 1, and p is 0; or
p is 1, x and q are 0, and y is 1;
and further wherein,
if $R_1$ is H then d is 0-8; or
if $R_1$ is not H then d is 0;

wherein $R_1$ is the side chain of an amino acid selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine;
wherein $R_2$ is selected from the group consisting of $NH_2$, NHR, $NR_2$, $NR_3{}^+H$, OH, SH, RO, RS, RSO, $RSO_2$, $CO_R$, CSR, COOH, COOR, $CONH_2$, CONHR, $CONR_2$, OCOR, and SCOR, wherein R = alkyl, alkenyl, aryl, aralkyl, or cycloalkyl;
wherein $R_3$ is H or $CH_{3;}$ and
wherein $R_x$ is a hydrophobic group;
or a pharmaceutically acceptable salt thereof.

**2.** The peptide according to claim 1, wherein $R_x$ comprises an aromatic carbon ring.

**3.** The peptide according to claim 2, wherein the aromatic ring comprises a 6- or 12 membered ring or a substituted form thereof.

**4.** The peptide according to claim 3, wherein the ring is substituted with at least one of: a lower alkyl, alkoxy, hydroxyl, carboxy, amine, thiol, hydrazide, amide, halide, hydroxyl, ether, amine, nitrile, imine, nitro, sulfide, sulfoxide, sulfone, thiol, aldehyde, keto, carboxy, ester, an amide group; a seleno group, a thio group and derivatives thereof.

**5.** The peptide according to claim 3, wherein the ring comprises between 1 and 5 substitutions.

**6.** The peptide according to claim 3, wherein the ring comprises 1 or 2 substitutions.

**7.** The peptide according to claim 2, wherein the aromatic carbon ring is selected from the group consisting of: a benzyl, phenyl, and naphthyl group.

**8.** The peptide according to claim 1 or claim 2, wherein the hydrophobic group is 6-membered aromatic carbon ring comprising a substituent at the 4-position.

**9.** A peptide for use in therapy according to claim 1 represented by the general formula II:

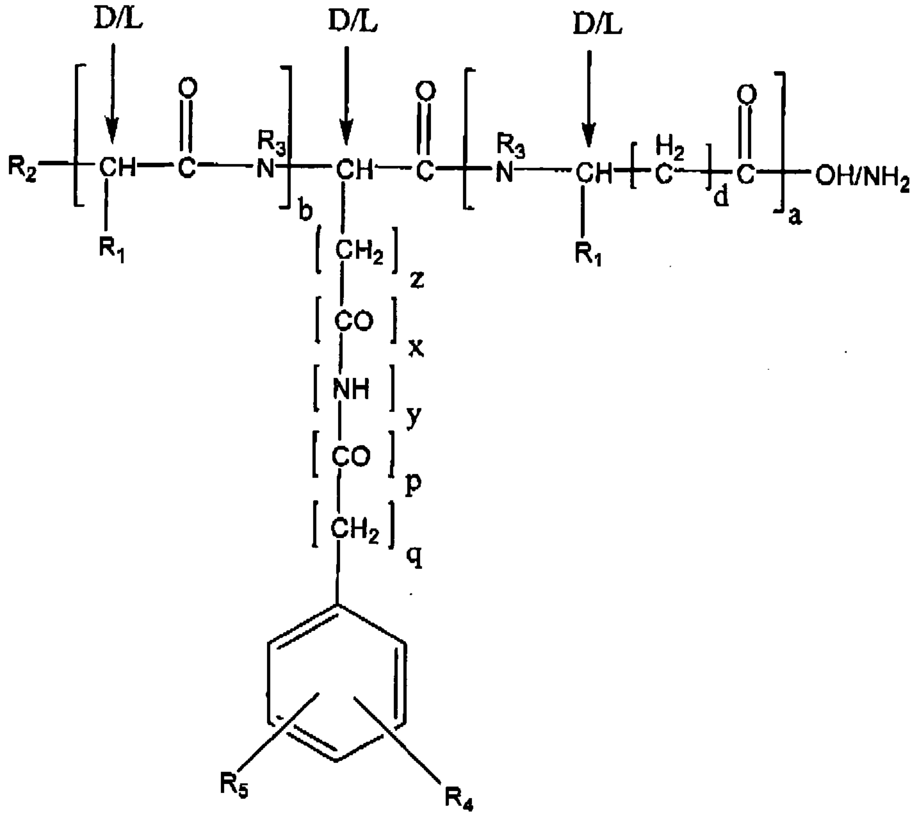

wherein:

a is 1 and b is 0; or
b is 1 and a is 0;
d is 0-8; and
z is 1-7; and
x is 1, y and q are 1, and p is 0; or
p is 1, x and q are 0, and y is 1;
and further wherein,
if $R_1$ is H then d is 0-8; or if $R_1$ is not H then d is 0;
wherein $R_1$ is the side chain of an amino acid selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine;
wherein $R_2$ is selected from the group consisting of $NH_2$, NHR, $NR_2$, $NR_3^+H$, OH, SH, RO, RS, RSO, $RSO_2$, $CO_R$. CSR, GOOH, COOR, $CONH_2$, CONHR, $CONR_2$, OCOR, and SCOR, wherein R = alkyl, alkenyl, aryl, aralkyl, or cycloalkyl;
wherein $R_3$ is H or $CH_{3;}$
wherein $R_4$ and $R_5$ are independently selected from the group consisting of H, alkyl, alkenyl, aryl, aralkyl, halogen, CN, $NO_2$, alkoxy, aryloxy, aralkyloxy, thioalkoxy, thioaryloxy, thioaralkyloxy, $+S(CH_3)_2$, $SO_3H$, $SO_2R$, $NH_2$, NHR, $NR_2$, $^+NR_3$, OH, SH, COOH, COOR, $CONH_2$, CONHR, $CONR_2$, $CH_2OH$, NCO, NCOR, NHOH, $NHNH_2$, NHNRH, $CH_2OCOR$, $CH_2OCSR$, COR, CSR, CSOR, $CF_3$, and $CCl_3$, and wherein R is alkyl, alkenyl, aryl, aralkyl, or cycloalkyl;
or a pharmaceutically acceptable salt thereof.

**10.** A peptide represented by the general formula I:

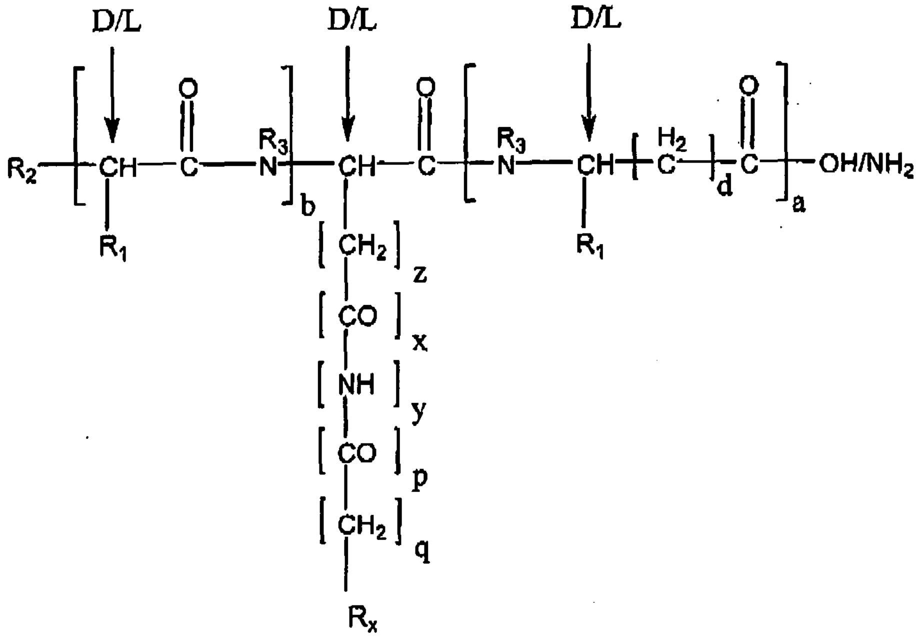

wherein:

a is 1 and b is 0; or
b is 1 and a is 0; and
d is 0-8; and
z is 1-7;
x is 1, y and q are 1, and p is 0; or
p is 1, x and q are 0, and y is 1;
and further wherein,
if $R_1$ is H then d is 0-8; or
if $R_1$ is not H then d is 0;
wherein $R_1$ is the side chain of an amino acid selected from the group consisting of alanine, arginine, asparagine, aspartic acid, cysteine, glutamic acid, glutamine, glycine, histidine, isoleucine, leucine, lysine, methionine, phenylalanine, proline, serine, threonine, tryptophan, tyrosine, and valine;
wherein $R_2$ is selected from the group consisting of $NH_2$, NHR, $NR_2$, $NR_3{}^+H$, OH, SH, RO, RS, RSO, $RSO_2$, $CO_R$, CSR, COOH, COOR, $CONH_2$, CONHR, CONR2, OCOR, and SCOR, wherein R = alkyl, alkenyl, aryl, aralkyl, or cycloalkyl;
wherein $R_3$ = H or $CH_3$; and
wherein $R_x$ is a 6-membered aromatic carbon ring comprising a substituent at the 4-position;
or a pharmaceutically acceptable salt thereof.

**11.** The peptide according to claim 10, wherein the aromatic carbon ring is substituted with at least one of: a lower alkyl, alkoxy, hydroxyl, carboxy, amine, thiol, hydrazide, amide, halide, hydroxyl, ether, amine, nitrile, imine, nitro, sulfide, sulfoxide, sulfone, thiol, aldehyde, keto, carboxy, ester, an amide group; a seleno group, a thio group and derivatives thereof.

**12.** The peptide according to any one of the preceding claims, wherein the peptide comprises a free N-terminal, a free C-terminal, or both a free N- and C-terminal.

**13.** The peptide of any one of the preceding claims, wherein the pharmaceutically acceptable salt is an acid addition salt, a metal salt, an ammonium salt or an amino acid addition salt.

**14.** The peptide of claim 13, wherein the pharmaceutically acceptable salt is a hydrochloride salt, a sulfate salt, a

phosphate salt, an acetate salt, a maleate salt, a fumarate salt, a tartrate salt, a citrate shalt, a sodium salt, a potassium salt, a magnesium salt, a calcium salt, an ammonium or tetramethyl ammonium salt,
a lysine salt, a glycine salt or a phenylalanine salt.

**15.** The peptide according to any one of the preceding claims, wherein the peptide further comprises a hydrogen bond group and the distance between the mass center of the hydrogen bond group and the hydrophobic group comprises from about 4 Angstroms to about 12 Angstrøms.

**16.** The peptide according to any one of the preceding claims, wherein the peptide further comprises a hydrogen bond group and the distance between the mass center of the hydrogen bond group and the hydrophobic group comprises from about 5 Angstrøms to about 10 Angstroms.

**17.** The peptide according to claim 10, wherein the substituent has a radius of from 3 to 11 Angstrøms.

**18.** The peptide according to claim 17, wherein the substituent is selected from the group consisting of a methyl, ethyl, t-butyl, c-hexyl, phenyl, n-butyl, n-hexyl, n-octyl, ethoxy, t-butoxy, phenoxy, butoxy, benzyloxy, n-hexyloxy, and n-octyloxy group.

**19.** The peptide according to any one of the preceding claims, wherein the peptide functions as an antiarrhythmic drug.

**20.** The peptide according to any one of the preceding claims, wherein the peptide is an orally available peptide.

**21.** The peptide according to any one of the preceding claims, wherein the peptide binds to an hPepT1 transporter or a biologically active fragment thereof.

**22.** The peptide according to any one of the preceding claims, wherein the peptide has a half-life in an *in vitro* plasma stability assay of more than 30 minutes.

**23.** The peptide according to any one of the preceding claims, wherein the peptide has a half-life in an *in vitro* plasma stability assay of more than about 48 hours.

**24.** The peptide according to any one of the preceding claims, wherein the peptide comprises a peptide bond that is modified to stabilize the peptide against enzymatic degradation.

**25.** The peptide according to any one of the preceding claims, wherein the peptide binds to a tissue, cell, or cell fraction that is a site of action for an antiarrhythmic peptide.

**26.** The peptide according to claim 23, wherein the antiarrhythmic peptide is selected from the group consisting of AAP, AAP10, HP5, or a functional analog thereof.

**27.** The peptide according to claim 23, wherein the peptide is a modulator of the function of the tissue, cell, or cell fraction.

**28.** The peptide according to claim 25, wherein the peptide antagonizes the function of the antiarrhythmic peptide.

**29.** The peptide according to claim 25, wherein the peptide agonizes the function of the antiarrhythmic.

**30.** The peptide according to claim 23, wherein the peptide is a modulator of a receptor of the antiarrhythmic peptide.

**31.** The peptide according to any one of the preceding claims, wherein the peptide is selected from the group consisting of the peptides shown in Table 1.

**32.** The peptide according to any one of the preceding claims, wherein the peptide is:

H-Gly-Lys(4-nitrobenzoyl)-OH (Compound 1);
H-Gly-Lys(4-methoxybenzoyl)-OH (Compound 4);
H-D-Lys(4-methoxybenzoyl)-Gly-OH (Compound 21);
H-$_D$-Lys(4-nitrobenzoyl)Gly-OH (Compound 22);
H-D-Lys(4-t-butylbenzoyl)-Gly-OH (Compound 54);

H-$_D$-Asn(NH(4-nitrobenzyl)Ala-OH (Compound 96);
H-$_D$-Lys(benzoyl)Gly-OH (Compound 23) or
H-$_D$-Asn(NH(4-methoxybenzyl)Ala-OH (Compound 95).

**33.** Use of a peptide according to any one of claims 1 to 32 in the manufacture of a medicament for the treatment of a pathological condition selected from a cardiovascular disease, inflammation of airway epithelium, a disorder of alveolar tissue, bladder incontinence, impaired hearing, an endothelial lesion, diabetic retinopathy, diabetic neuropathy, ischemia of the central nervous system, ischemia of the spinal cord, a dental tissue disorder, kidney disease, failure of bone marrow transplantation, wound, erectile dysfunction, urinary bladder incontinence, neuropathic pain, subchronic and chronic inflammation, cancer, transplantation failure, osteoporosis or a pathology affecting bone formation, growth or maintenance

**34.** The use according to claim 33, wherein the cardiovascular disease is selected from arrhythmia, acute ischemic heart disease, congestive heart failure, congenital heart diseases, cor pulmonale, cardiomyopathy, myocarditis or hypertensive heart disease.

**35.** The use according to claim 33 or claim 34, wherein the medicament is for oral administration.

**36.** The use according to any one of claims 33 to 35, wherein the medicament is for administration to a human patient.

## Patentansprüche

**1.** Peptid zur therapeutischen Verwendung, dargestellt durch die allgemeine Formel I:

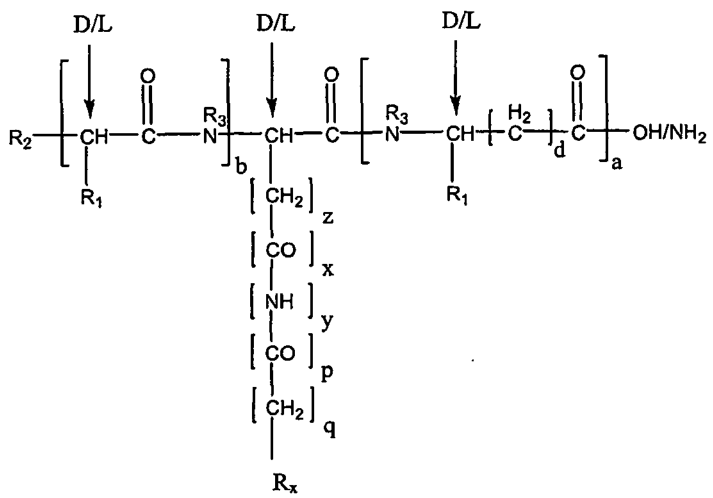

worin:

a = 1 ist und b = 0 ist; oder
b = 1 ist und a = 0 ist;
d = 0 bis 8 ist; und
z = 1 bis 7 ist; und
x = 1 ist, y und q = 1 sind und p = 0 ist; oder
p = 1 ist, x und q = 0 sind und y = 1 ist;
und worin weiters,
wenn $R_1$ = H ist, d = 0 bis 8 ist; oder,
wenn $R_1$ nicht H ist, d = 0 ist;
worin $R_1$ die Seitenkette einer aus der aus Alanin, Arginin, Asparagin, Asparaginsäure, Cystein, Glutaminsäure, Glutamin, Glycin, Histidin, Isoleucin, Leucin, Lysin, Methionin, Phenylalanin, Prolin, Serin, Threonin, Tryptophan, Tyrosin und Valin bestehenden Gruppe ausgewählten Aminosäure ist;

worin $R_2$ aus der aus $NH_2$, NHR, $NR_2$, $NR_3{}^+H$, OH, SH, RO, RS, RSO, $RSO_2$ COR, CSR, COOH, COOR, $CONH_2$, CONHR, $CONR_2$, OCOR und SCOR bestehenden Gruppe ausgewählt ist und worin R = Alkyl, Alkenyl, Aryl, Aralkyl oder Cycloalkyl ist;
worin $R_3$ = H oder $CH_3$ ist; und
worin $R_x$ eine hydrophobe Gruppe ist;
oder ein pharmazeutisch annehmbares Salz davon.

**2.** Peptid nach Anspruch 1, worin $R_x$ einen aromatischen Kohlenstoffring umfasst.

**3.** Peptid nach Anspruch 2, worin der aromatische Ring einen 6- oder 12-gliedrigen Ring oder eine substituierte Form davon umfasst.

**4.** Peptid nach Anspruch 3, worin der Ring mit zumindest einem von einem Niederalkyl, Alkoxy, Hydroxyl, Carboxy, Amin, Thiol, Hydrazid, Amid, Halogenid, Hydroxyl, Ether, Amin, Nitril, Imin, Nitro, Sulfid, Sulfoxid, Sulfon, Thiol, Aldehyd, Keto, Carboxy, Ester, einer Amidgruppe, einer Selenogruppe, einer Thiogruppe und Derivaten davon substituiert ist.

**5.** Peptid nach Anspruch 3, worin der Ring zwischen 1 und 5 Substitutionen umfasst.

**6.** Peptid nach Anspruch 3, worin der Ring 1 oder 2 Substitutionen umfasst.

**7.** Peptid nach Anspruch 2, worin der aromatische Kohlenstoffring aus der aus einer Benzyl-, einer Phenyl- und einer Naphthylgruppe bestehenden Gruppe ausgewählt ist.

**8.** Peptid nach Anspruch 1 oder Anspruch 2, worin die hydrophobe Gruppe ein 6-gliedriger aromatischer Kohlenstoffring ist, der einen Substituenten an Position 4 umfasst.

**9.** Peptid zur therapeutischen Verwendung nach Anspruch 1, dargestellt durch die allgemeine Formel II:

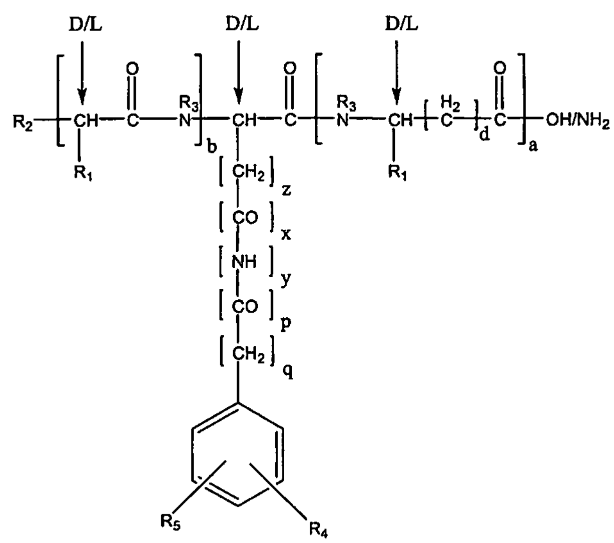

worin:

a = 1 ist und b = 0 ist; oder
b = 1 ist und a = 0 ist;
d = 0 bis 8 ist; und
z = 1 bis 7 ist; und
x = 1 ist, y und q = 1 sind und p = 0 ist; oder
p = 1 ist, x und q = 0 sind und y = 1 ist;
und worin weiters,

wenn $R_1$ = H ist, d = 0 bis 8 ist; oder,
wenn $R_1$ nicht H ist, d = 0 ist;
worin $R_1$ die Seitenkette einer aus der aus Alanin, Arginin, Asparagin, Asparaginsäure, Cystein, Glutaminsäure, Glutamin, Glycin, Histidin, Isoleucin, Leucin, Lysin, Methionin, Phenylalanin, Prolin, Serin, Threonin, Tryptophan, Tyrosin und Valin bestehenden Gruppe ausgewählten Aminosäure ist;
worin $R_2$ aus der aus $NH_2$, NHR, $NR_2$, $NR_3{}^+H$, OH, SH, RO, RS, RSO, $RSO_2$, $CO_R$, CSR, COOH, COOR, $CONH_2$, CONHR, $CONR_2$, OCOR und SCOR bestehenden Gruppe ausgewählt ist und worin R = Alkyl, Alkenyl, Aryl, Aralkyl oder Cycloalkyl ist;
worin $R_3$ = H oder $CH_3$ ist; und
worin $R_4$ und $R_5$ unabhängig voneinander aus der aus H, Alkyl, Alkenyl, Aryl, Aralkyl, Halogen, CN, $NO_2$, Alkoxy, Aryloxy, Aralkyloxy, Thioalkoxy, Thioaryloxy, Thioaralkyloxy, $^+S(CH_3)_2$, $SO_3H$, $SO_2R$, $NH_2$, NHR, $NR_2$, $^+NR_3$, OH, SH, COOH, COOR, $CONH_2$, CONHR, $CONR_2$, $CH_2OH$, NCO, NCOR, NHOH, $NHNH_2$, NHNRH, $CH_2OCOR$, $CH_2OCSR$, COR, CSR, CSOR, $CF_3$ und $CCl_3$ bestehenden Gruppe ausgewählt sind und worin R Alkyl, Alkenyl, Aryl, Aralkyl oder Cycloalkyl ist; oder ein pharmazeutisch annehmbares Salz davon.

**10.** Peptid, dargestellt durch die allgemeine Formel I:

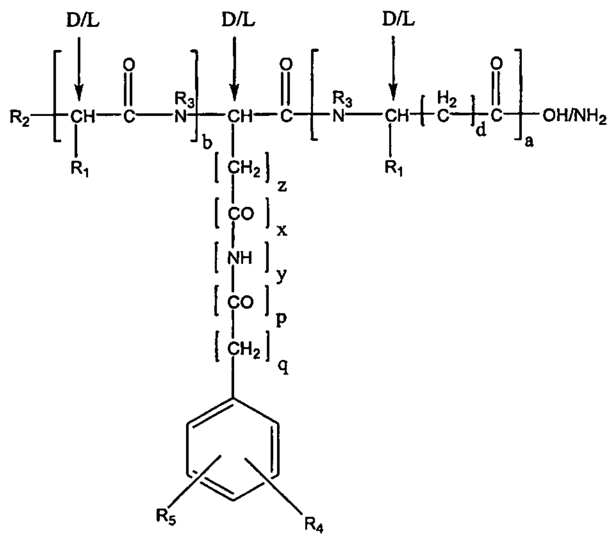

worin:

a = 1 ist und b = 0 ist; oder
b = 1 ist und a = 0 ist; und
d = 0 bis 8 ist; und
z = 1 bis 7 ist;
x = 1 ist, y und q = 1 sind und p = 0 ist; oder
p = 1 ist, x und q = 0 sind und y = 1 ist;
und worin weiters,
wenn $R_1$ = H ist, d = 0 bis 8 ist; oder,
wenn $R_1$ nicht H ist, d = 0 ist;
worin $R_1$ die Seitenkette einer aus der aus Alanin, Arginin, Asparagin, Asparaginsäure, Cystein, Glutaminsäure, Glutamin, Glycin, Histidin, Isoleucin, Leucin, Lysin, Methionin, Phenylalanin, Prolin, Serin, Threonin, Tryptophan, Tyrosin und Valin bestehenden Gruppe ausgewählten Aminosäure ist;
worin $R_2$ aus der aus $NH_2$, NHR, $NR_2$, $NR_3{}^+H$, OH, SH, RO, RS, RSO, $RSO_2$, COR, CSR, COOH, COOR, $CONH_2$, CONHR, $CONR_2$, OCOR und SCOR bestehenden Gruppe ausgewählt ist und worin R = Alkyl, Alkenyl, Aryl, Aralkyl oder Cycloalkyl ist;
worin $R_3$ = H oder $CH_3$ ist; und
worin $R_x$ ein 6-gliedriger aromatischer Kohlenstoffring ist, der einen Substituenten an Position 4 umfasst; oder ein pharmazeutisch annehmbares Salz davon.

**11.** Peptid nach Anspruch 10, worin der aromatische Kohlenstoffring mit zumindest einem von einem Niederalkyl, Alkoxy, Hydroxyl, Carboxy, Amin, Thiol, Hydrazid, Amid, Halogenid, Hydroxyl, Ether, Amin, Nitril, Imin, Nitro, Sulfid, Sulfoxid, Sulfon, Thiol, Aldehyd, Keto, Carboxy, Ester, einer Amidgruppe, einer Selenogruppe, einer Thiogruppe und Derivaten davon substituiert ist.

**12.** Peptid nach einem der vorangegangenen Ansprüche, worin das Peptid einen freien N-Terminus, einen freien C-Terminus oder sowohl einen freien N- als auch einen freien C-Terminus umfasst.

**13.** Peptid nach einem der vorangegangenen Ansprüche, woran das pharmazeutisch annehmbare Salz ein Säureadditionssalz, ein Metallsalz, ein Ammoniumsalz oder ein Aminosäureadditionssalz ist.

**14.** Peptid nach Anspruch 13, worin das pharmazeutisch annehmbare Salz ein Hydrochloridsalz, ein Sulfatsalz, ein Phosphatsalz, ein Acetatsalz, ein Maleatsalz, ein Fumaratsalz, ein Tartratsalz, ein Citratsalz, ein Natriumsalz, ein Kaliumsalz, ein Magnesiumsalz, ein Calciumsalz, ein Ammonium- oder Tetramethylammoniumsalz, ein Lysinsalz, ein Glycinsalz oder ein Phenylalaninsalz ist.

**15.** Peptid nach einem der vorangegangenen Ansprüche, worin das Peptid weiters eine Wasserstoffbindungsgruppe umfasst und die Entfernung zwischen dem Massenmittelpunkt der Wasserstoffbindungsgruppe und der hydrophoben Gruppe etwa 4 Angström bis etwa 12 Angström umfasst.

**16.** Peptid nach einem der vorangegangenen Ansprüche, worin das Peptid weiters eine Wasserstoffbindungsgruppe umfasst und die Entfernung zwischen dem Massenmittelpunkt der Wasserstoffbindungsgruppe und der hydrophoben Gruppe etwa 5 Angström bis etwa 10 Angström umfasst.

**17.** Peptid nach Anspruch 10, worin der Substituent einen Radius von 3 bis 11 Angström aufweist.

**18.** Peptid nach Anspruch 17, worin der Substituent aus der aus einer Methyl-, Ethyl-, t-Butyl-, c-Hexyl-, Phenyl-, n-Butyl-, n-Hexyl-, n-Octyl-, Ethoxy-, t-Butoxy-, Phenoxy-, Butoxy-, Benzyloxy-, n-Hexyloxy- und n-Octyloxy-Gruppe bestehenden Gruppe ausgewählt ist.

**19.** Peptid nach einem der vorangegangenen Ansprüche, worin das Peptid als antiarrhythmisches Arzneimittel fungiert.

**20.** Peptid nach einem der vorangegangenen Ansprüche, worin das Peptid ein oral verfügbares Peptid ist.

**21.** Peptid nach einem der vorangegangenen Ansprüche, worin das Peptid an einen hPepT1-Transporter oder ein biologisch aktives Fragment davon bindet.

**22.** Peptid nach einem der vorangegangenen Ansprüche, worin das Peptid in einem In-vitro-Plasmastabilitätstest eine Halbwertszeit von mehr als 30 min aufweist.

**23.** Peptid nach einem der vorangegangenen Ansprüche, worin das Peptid in einem In-vitro-Plasmastabilitätstest eine Halbwertszeit von mehr als 48 h aufweist.

**24.** Peptid nach einem der vorangegangenen Ansprüche, worin das Peptid eine Peptidbindung umfasst, die so modifiziert ist, dass sie das Peptid gegen enzymatischen Abbau stabilisiert.

**25.** Peptid nach einem der vorangegangenen Ansprüche, worin das Peptid an ein Gewebe, eine Zelle oder eine Zellfraktion bindet, das bzw. die eine Wirkstelle für ein antiarrythmisches Peptid ist.

**26.** Peptid nach Anspruch 23, worin das antiarrhythmische Peptid aus der aus AAP, AAP10, HP5 und einem funktionellen Analog davon bestehenden Gruppe ausgewählt ist.

**27.** Peptid nach Anspruch 25, worin das Peptid ein Modulator der Funktion des Gewebes, der Zelle oder der Zellfraktion ist.

**28.** Peptid nach Anspruch 25, worin das Peptid der Funktion des antiarrhythmischen Peptids entgegenwirkt.

**29.** Peptid nach Anspruch 25, worin das Peptid die Funktion des antiarrhythmischen Peptids unterstützt.

30. Peptid nach Anspruch 23, worin das Peptid ein Modulator eines Rezeptors des antiarrhythmischen Peptids ist.

31. Peptid nach einem der vorangegangenen Ansprüche, worin das Peptid aus der aus den in Tabelle 1 gezeigten Peptiden bestehenden Gruppe ausgewählt ist.

32. Peptid nach einem der vorangegangenen Ansprüche, worin das Peptid:

H-Gly-Lys(4-nitrobenzoyl)-OH (Verbindung 1);
H-Gly-Lys(4-methoxybenzoyl)-OH (Verbindung 4);
H-D-Lys(4-methoxybenzoyl)Gly-OH (Verbindung 21);
H-D-Lys(4-nitrobenzoyl)Gly-OH (Verbindung 22);
H-D-Lys(4-t-butylbenzoyl)Gly-OH (Verbindung 54);
H-D-Asn(NH(4-nitrobenzoyl))Ala-OH (Verbindung 96);
H-D-Lys(benzoyl)Gly-OH (Verbindung 23) oder
H-D-Asn(NH(4-methoxybenzyl))Ala-OH (Verbindung 95) ist.

33. Verwendung eines Peptids nach einem der Ansprüche 1 bis 32 bei der Herstellung eines Medikaments zur Behandlung eines aus einer Herz-Gefäß-Erkrankung, einer Entzündung des Luftröhrenepithels, einer Erkrankung des Alveolargewebes, Blaseninkontinenz, Gehörschaden, einer Endothelläsion, diabetischer Retinopathie, diabetischer Neuropathie, Ischämie des Zentralnervensystems, Ischämie des Rückenmarks, einer Zahngewebeerkrankung, Nierenerkrankung, gescheiterter Knochenmarktransplantation, einer Wunde, erektiler Dysfunktion, Harnblaseninkontinenz, neuropathischem Schmerz, subchronischer und chronischer Entzündung, Krebs, gescheiterter Transplantation, Osteoporose oder einer Pathologie, die die Bildung, das Wachstum oder die Erhaltung des Knochens betrifft, ausgewählten pathologischen Leidens.

34. Verwendung nach Anspruch 33, worin die Herz-Gefäß-Erkrankung aus Arrythmie, akuter ischämischer Herzerkrankung, kongestiver Herzinsuffizienz, kongenitalen Herzerkrankungen, Cor pulmonale, Myokarditis oder hypertonischer Herzerkrankung ausgewählt ist.

35. Verwendung nach Anspruch 33 oder Anspruch 34, worin das Medikament zur oralen Verabreichung dient.

36. Verwendung nach einem der Ansprüche 33 bis 35, worin das Medikament zur Verabreichung an einen menschlichen Patienten dient.

**Revendications**

1. Peptide pour utilisation en thérapie représenté par la formule générale I:

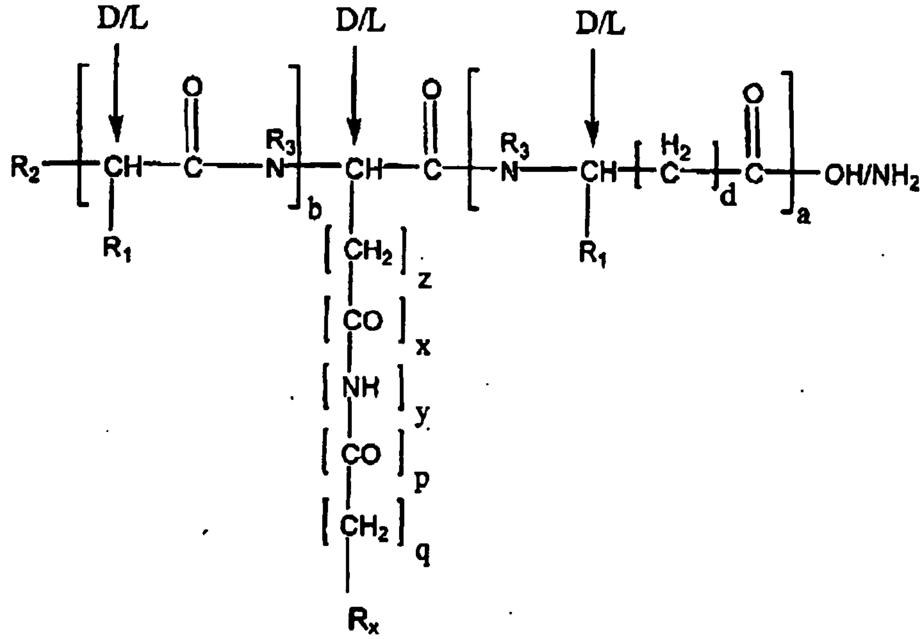

où:

a est 1 et b est 0; ou
b est 1 et a est 0;
d est 0-8; et
z est 1-7; et
x est 1, y et q sont 1, et p est 0; ou
p est 1, x et q sont 0, et y est 1;
et en outre où,
si $R_1$ est H, alors d est 0-8; ou
si $R_1$ n'est pas H, alors d est 0;
où $R_1$ est la chaîne latérale d'un acide aminé sélectionné dans le groupe consistant en alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine et valine;
où $R_2$ est sélectionné dans le groupe consistant en $NH_2$, NHR, $NR_2$, $NR_3^+H$, OH, SN, RO, RS, RSO, $RSO_2$, COR, CSR, COOH, COOR, $CONH_2$, CONHR, CONR2, OCOR et SCOR, où R = alkyle, alkényle, aryle, aralkyle ou cycloalkyle;
où $R_3$ est H ou $CH_3$ et
où $R_x$ est un groupe hyrophobe;
ou un sel pharmaceutiquement acceptable de celui-ci.

**2.** Peptide selon la revendication 1, où $R_x$ comprend un cycle de carbone aromatique.

**3.** Peptide selon la revendication 2, où le cycle aromatique comprend un cycle à 6 ou 12 membres ou une forme substituée de celui-ci.

**4.** Peptide selon la revendication 3, où le cycle est substitué par au moins un de: alkyle inférieur, alkoxy, hydroxyle, carboxy, amine, thiole, hydrazide, amide, halogénure, hydroxyle, éther, amine, nitrile, imine, nitro, sulfure, sulfoxyde, sulfone, thiol, aldéhyde, kéto, carboxy, ester, un groupe amide; un groupe séléno; un groupe thio et leurs dérivés.

**5.** Peptide selon la revendication 3, où le cycle comprend entre 1 et 5 substitutions.

**6.** Peptide selon la revendication 3, où le cycle comprend 1 ou 2 substitutions.

**7.** Peptide selon la revendication 2, où le cycle de carbone aromatique est sélectionné dans le groupe consistant en: un groupe benzyle, phényle et naphtyle.

**8.** Peptide selon la revendication 1 ou la revendication 2, où le groupe hydrophobe est un cycle de carbone aromatique à 6 membres comprenant un substituant à la position 4.

**9.** Peptide pour utilisation en thérapie selon la revendication 1, représenté par la formule générale II:

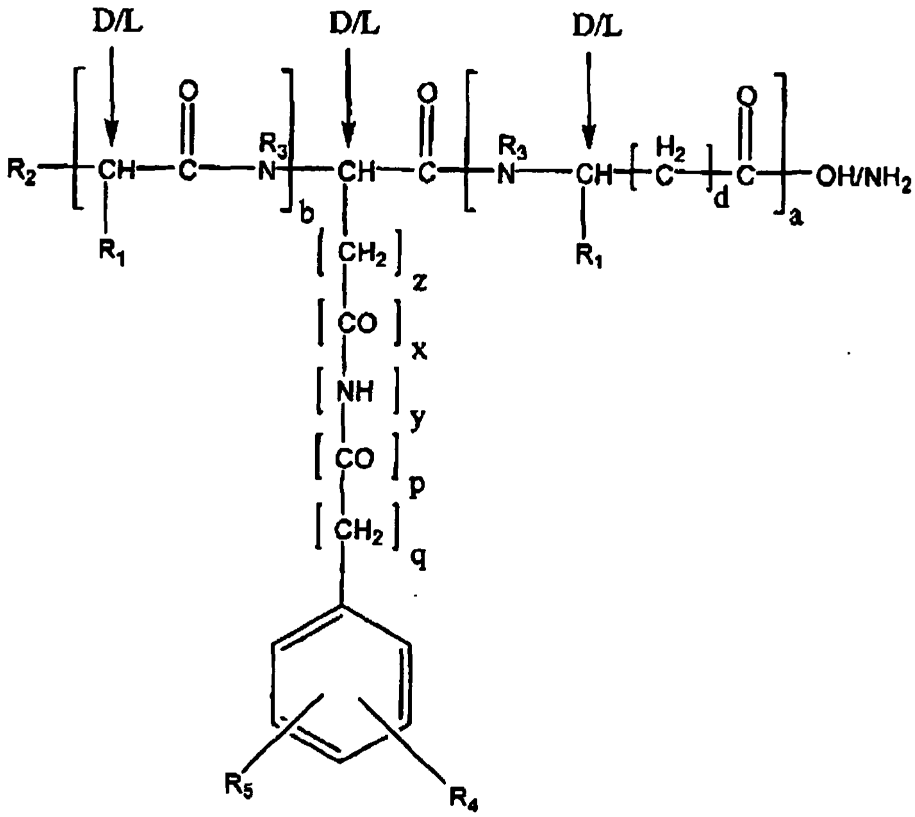

où:

a est 1 et b est 0; ou
b est 1 et a est 0;
d est 0-8; et
z est 1-7; et
x est 1, y et q sont 1, et p est 0; ou
p est 1, x et q sont 0, et y est 1;
et en outre où:

si $R_1$ est H, alors d est 0-8; ou
si $R_1$ n'est pas H, alors d est 0;
où $R_1$ est la chaîne latérale d'un acide aminé sélectionné dans le groupe consistant en alanine, arginine, asparagine, acide aspartique, cystéine, acide glutamique, glutamine, glycine, histidine, isoleucine, leucine, lysine, méthionine, phénylalanine, proline, serine, threonine, tryptophan, tyrosine et valine;
où $R_2$ est sélectionné dans le groupe consistant en $NH_2$,
NHR, $NR_2$, $NR_3{}^+H$, OH, SH, RO, RS, RSO, $RSO_2$, $CO_R$, CSR, COOH, COOR, $CONH_2$, CONHR, $CONR_2$, OCOR et SCOR, où R = alkyle, alkényle, aryle, aralkyle ou cycloalkyle:

où $R_3$ is H or $CH_3$;

où $R_4$ et $R_5$ sont indépendamment sélectionnés dans le groupe consistant en H, alkyle, alkényle, aryle, aralkyle, halogène, CN, $NO_2$, alkoxy, aryloxy, aralkyloxy, thioalkoxy, thioaryloxy, thioaralkyloxy, $+S(CH_3)_2$, $SO_3H$, $SO_2R$, $NH_2$, NHR, $NR_2$, $+NR_3$, OH, SH, COOH, COOR, $CONH_2$, CONHR, $CONR_2$, $CH_2OH$,.
NCO, NCOR, NHOH, $NHNH_2$, NHNRH, $CH_2OCOR$, $CH_2OCSR$, COR, CSR, CSOR, $CF_3$ et $CCl_3$, et où R est alkyle, alkényle, aryle, aralkyle ou cycloalkyle;
où un sel pharmaceutiquement acceptable de celui-ci.

**10.** Peptide représenté par la formule générale I:

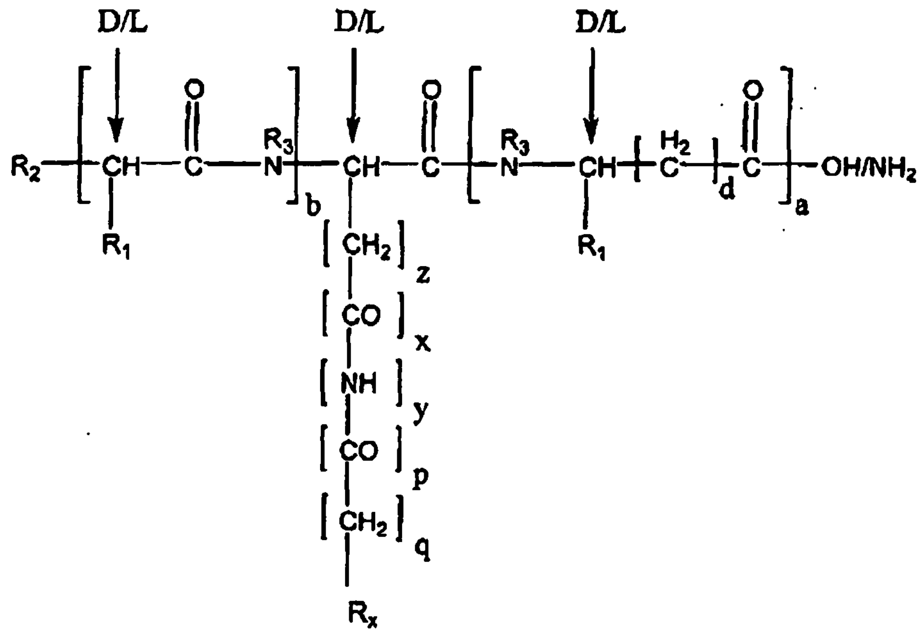

où
a est 1 et b est 0; ou b est 1 et a est 0; et d est 0-8; et
z est 1-7;
x est 1, y et q sont 1, et p est 0; ou
p est 1, x et q sont 0, et y est 1;
et en outre où,
si $R_1$ est H alors d est 0-8; ou
si $R_1$ n'est pas H, alors d est 0;
où $R_1$ est la chaîne latérale d'un acide amine sélectionné dans le groupe consistant en alanine, arginine, asparagine, acide aspartique, cystéine, acide glutamique, glutamine, glycine, histidine, isoleucine, leucine, lysine, méthionine, phénylalanine, proline, sérine, thréonine, tryptophane, tyrosine et valine;
où $R_2$ est sélectionné dans le groupe consistant en $NH_2$,
NHR, $NR_2$, $NR_3$+H, OH, SH, RO, RS, RSO, $RSO_2$, $CO_R$, CSR, COOH, COOR, $CONH_2$, CONHR, CONR2, OCOR et SCOR, où R = alkyle, alkényle, aryle, aralkyle ou cycloalkyle;
où $R_3$ = H ou $CH_3$; et
où $R_x$ est un cycle de carbone aromatique à 6 membres comprenant un substituant à la positon 4;
ou un sel pharmaceutiquement acceptable de celui-ci.

**11.** Peptide selon la revendication 10, où le cycle de carbone aromatique est substitué par au moins un de: un alkyle inférieur, alkoxy, hydroxyle, carboxy, amine, thiol, hydrazide, amide, halogénure, hydroxyl, éther, amine, nitrile, imine, nitro, sulfure, sulfoxide, sulfane, thiol, aldéhyde, kéto, carboxy, ester, un groupe amide; un groupe séléno, un groupe thio et leurs dérivés.

**12.** Peptide selon l'une quelconque des revendications précédentes, où le peptide comprend un terminal N-libre, un terminal-C libre, ou à la fois un terminal N- et un terminal-C libre.

**13.** Peptide selon l'une quelconque des revendications précédentes, où le sel pharmaceutiquement acceptable est un sel d'addition d'acide, un sel métallique, un sel d'ammonium ou un sel d'addition d'acide aminé.

**14.** Peptide selon la revendication 13, où le sel pharmaceutiquement acceptable est un sel de chlorhydrate, un sel de sulfate, un sel de phosphate, un sel d'acétate, un sel de maléate, un sel de fumarate, un sel de tartrate, un sel de citrate, un sel de sodium, un sel de potassium, un sel de magnésium, un sel de calcium, un sel d'ammonium ou de tétraméthyl d'ammonium, un sel de lysine, un sel de glycine ou un sel de phénylalanine.

**15.** Peptide selon l'une quelconque des revendications précédentes, où le peptide comprend en outre un groupe de liaison d'hydrogène, et la distance entre le centre de masse du groupe de liaison d'hydrogène et le groupe hydrophobe comprend entre environ 4 Angstrøms à environ 12 Angstrøms.

**16.** Peptide selon l'une quelconque des revendications précédentes, où le peptide comprend en outre un groupe de liaison d'hydrogène, et la distance entre le centre de masse du groupe de liaison d'hydrogène et le groupe hydrophobe comprend d'environ 5 Angstrøms à environ 10 Angstrøms.

**17.** Peptide selon la revendication 10, où le substituant a un rayon de 3 à 11 Angstrøms.

**18.** Peptide selon la revendication 17, où le substituant est sélectionné dans le groupe consistant en un groupe méthyle, éthyle, t-butyle, c-hexyle, phényle, n-butyle, n-hexyle, n-octyle, éthoxy, t-butoxy, phénoxy, butoxy, benzyloxy, n-hexyloxy et un groupe n-octyloxy.

**19.** Peptide selon l'une quelconque des revendications précédentes, où le peptide fonctionne comme médicament antiarythmique.

**20.** Peptide selon l'une quelconque des revendications précédentes, où le peptide est un peptide disponible oralement.

**21.** Peptide selon l'une quelconque des revendications précédentes, où le peptide se lie à un transporteur de hPepT1 ou un fragment biologiquement actif de celui-ci.

**22.** Peptide selon l'une quelconque des revendications précédentes, où le peptide a une demi-vie dans un essai de stabilité de plasma *in vitro* de plus de 30 minutes.

**23.** Peptide selon l'une quelconque des revendications précédentes, où le peptide a une demi-vie dans un essai de stabilité de plasma *in vitro* de plus qu'environ 48 heures.

**24.** Peptide selon l'une quelconque des revendications précédentes, où le peptide comprend une liaison de peptide qui est modifiée pour stabiliser le peptide à l'encontre d'une dégradation enzymatique.

**25.** Peptide selon l'une quelconque des revendications précédentes, où le peptide se lie à un tissu, cellule ou fraction de cellule qui est un site d'action pour un peptide antiarythmique.

**26.** Peptide selon la revendication 23, où le peptide antiarythmique est sélectionné dans le groupe consistant en AAP, AAP10, HP5 ou un analogue fonctionnel de ceux-ci.

**27.** Peptide selon la revendication 23, où le peptide est un modulateur de la fonction du tissu, de la cellule ou de la fraction de cellule.

**28.** Peptide selon la revendication 25, où le peptide antagonise la fonction du peptide antiarythmique.

**29.** Peptide selon la revendication 25, où le peptide agonise la fonction de l'antiarythmique.

**30.** Peptide selon la revendication 23, où le peptide est un modulateur d'un récepteur du peptide antiarythmique.

**31.** Peptide selon l'une quelconque des revendications précédentes, où le peptide est sélectionné dans le groupe consistant en peptides indiqués dans le Tableau 1.

**32.** Peptide selon l'une quelconque des revendications précédentes, où le peptide est:

H-Gly-Lys(4-nitrobenzoyl)-OH (Composé 1);
H-Gly-Lys(4-méthoxybenzoyl)-OH (Composé 4);
H-D-Lys(4-méthoxybenzoyl)-Gly-OH (Composé 21);
$H-_D$-Lys(4-nitrobenzoyl)Gly-OH (Composé 22);
H-D-Lys(4-t-butylbenzoyl)-Gly-OH (Composé 54);
$H-_D$-Asn(NH(4-nitrobenzyl)Ala-OH (Composé 96);
$H-_D$-Lys(benzoyl)Gly-OH (Composé 23) ou
$H-_D$-Asn(NH(4-methoxybenzyl)Ala-OH (Composé 95).

**33.** Utilisation d'un peptide selon l'une quelconque des revendications 1 à 32 dans la fabrication d'un médicament pour le traitement d'un état pathologique sélectionné parmi une maladie cardiovasculaire, l'inflammation de l'épithélium des voies respiratoires, une maladie du tissu alvéolaire, l'incontinence de la vessie, altération auditive, une lésion endothéliale, la rétinopathie diabétique, la neuropathie diabétique, l'ischémie du système nerveux central, l'ischémie de la moelle épinière, un trouble du tissu dentaire, une maladie des reins, un échec d'une transplantation de moelle osseuse, plaie, troubles d'érection, incontinence de vessie urinaire, douleur neuropathique, inflammation sous-chronique et chronique, cancer, échec de transplantation, ostéoporose ou une pathologie affectant la formation, croissance ou conservation de l'os.

**34.** Utilisation selon la revendication 33, où la maladie cardiovasculaire est sélectionnée parmi l'arythmie, la cardiopathie ischémique aiguë, l'insuffisance cardiaque congestive, les maladies de coeur congénitales, coeur pulmonaire, cardiomyopathie, myocardite ou cardiopathie due à l'hypertension artérielle.

**35.** Utilisation selon la revendication 33 ou la revendication 34, où le médicament est pour l'administration orale.

**36.** Utilisation selon l'une quelconque des revendications 33 à 35, où le médicament est pour l'administration à un patient humain.

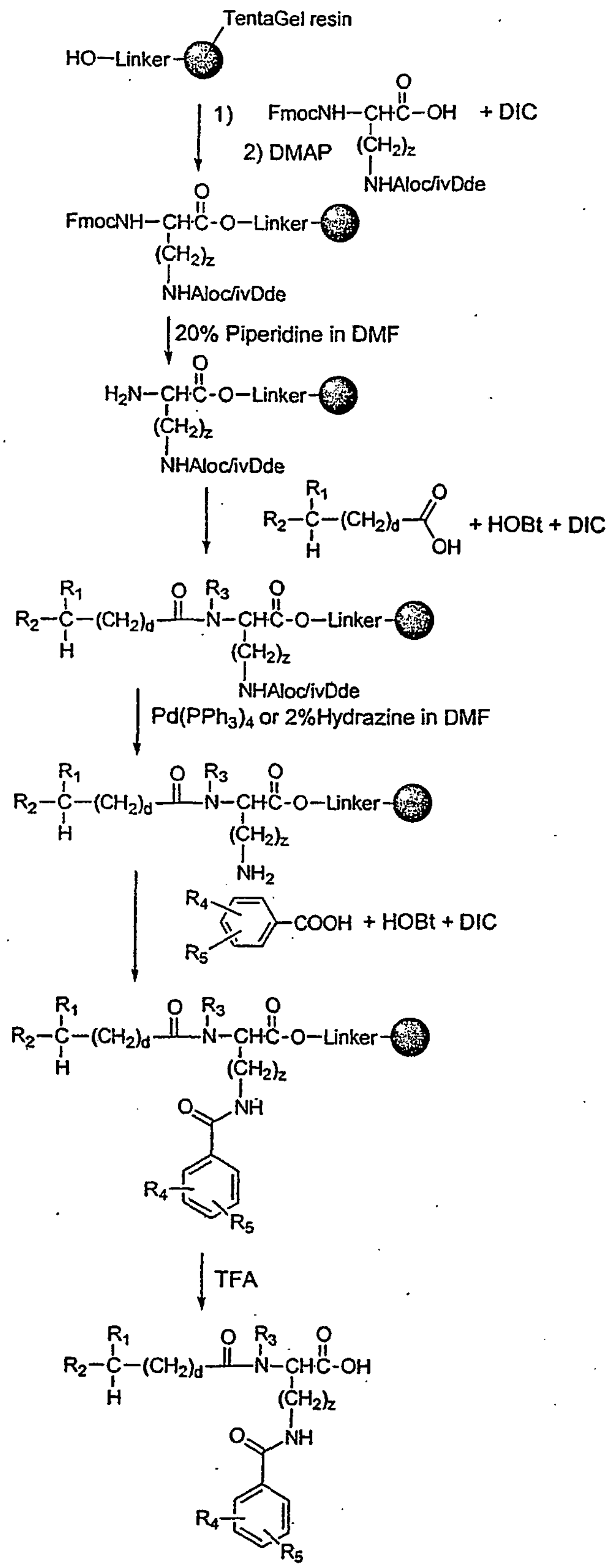
TentaGel resin
1)
+ DIC
2) DMAP
20% Piperidine in DMF
+ HOBt + DIC
Pd(PPh3)4 or 2%Hydrazine in DMF
+ HOBt + DIC
TFA

**Figure 1a**

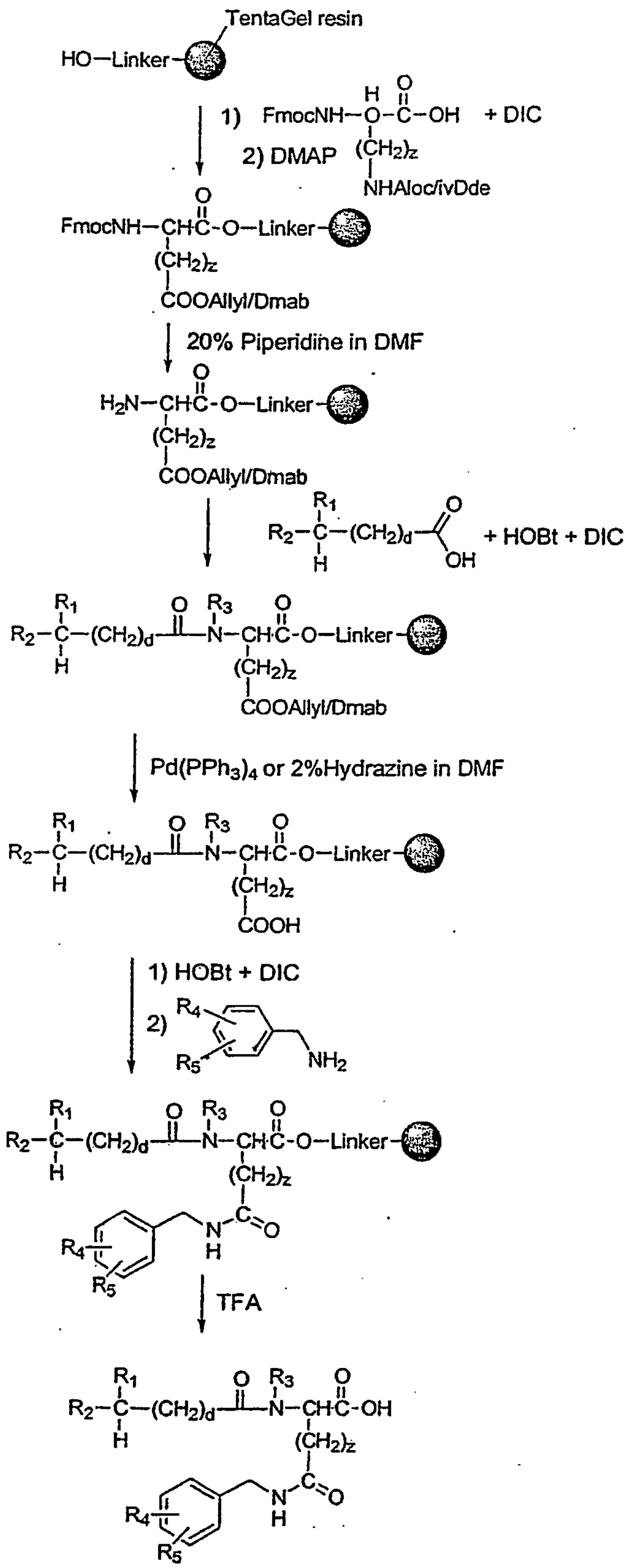
TentaGel resin
1) FmocNH + DIC
2) DMAP
20% Piperidine in DMF
+ HOBt + DIC
Pd(PPh3)4 or 2%Hydrazine in DMF
1) HOBt + DIC
2)
TFA

Figure 1b

Figure 2

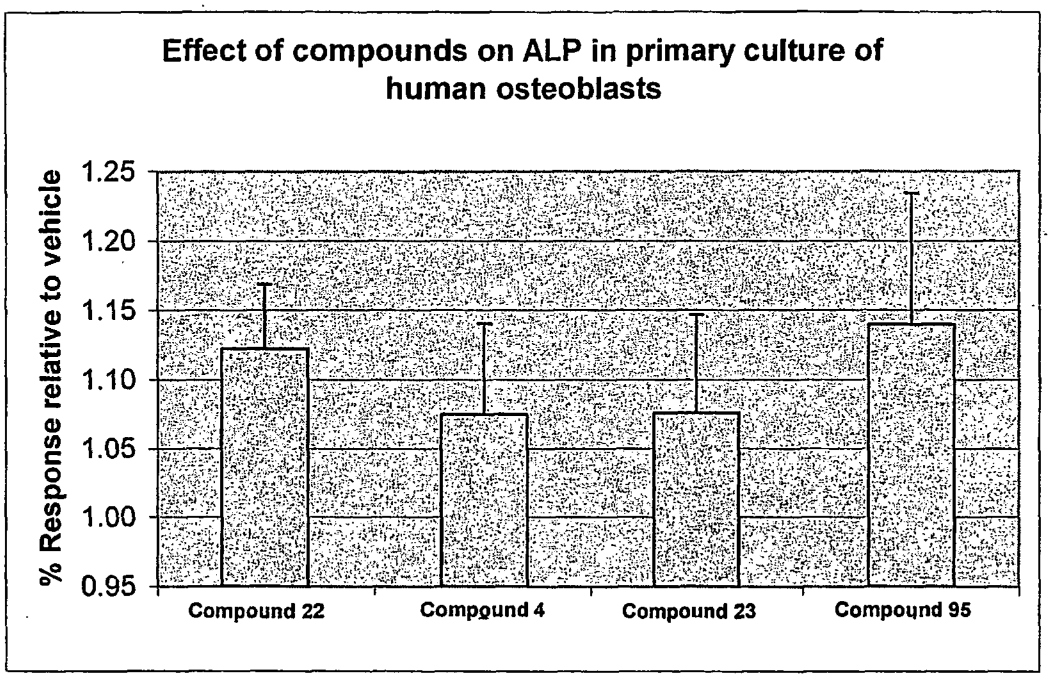
Effect of compounds on ALP in primary culture of human osteoblasts
% Response relative to vehicle
1.25
1.20
1.15
1.10
1.05
1.00
0.95
Compound 22
Compound 4
Compound 23
Compound 95

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02077017 A
- US 0205773 W
- WO 2002077017 A
- US 5350741 A
- WO 9811125 A


### Non-patent literature cited in the description


- **Sperelakis, N.** Cell Interactions and Gap Junctions. 1989
- **Kaprielian, R. R. et al.** *Circulation,* 1998, vol. 97, 651-660
- **Saffitz, J. E. et al.** *Cardiovasc Res.,* 1999, vol. 42, 309-317
- **Aonuma, S. et al.** *Chem Pharm Bull (Tokyo),* 1980, vol. 28, 3332-3339
- **Aonuma, S. et al.** *Chem Pharm Bull (Tokyo),* 1980, vol. 28, 3340-3346
- **Ronsberg, M.A. et al.** *Med. Sci.,* 1986, vol. 14, 350-351
- **Aonuma, S. et al.** *J Pharmacobiodyn.,* 1982, vol. 5, 40-48
- **Dikshit, M. et al.** *Indian J. Exp. Biol.,* 1988, vol. 26, 874-876
- **Kohama, Y. et al.** *Chem. Pharm. Bull. (Tokyo),* 1987, vol. 35, 3928-3930
- **Kohama, Y. et al.** *Chem. Pharm. Bull. (Tokyo),* 1988, vol. 36, 4597-4599
- **Dhein, S. et al.** *Naunyn Schmiedebergs Arch Pharmacol.,* 1994, vol. 350, 174-184
- **Muller, A. et al.** *Eur. J. Pharmacol.,* 1997, vol. 327, 65-72
- **Bailey, P.D. et al.** *Angew. Chem. Int. Ed.,* 2000, vol. 39, 506
- **Seki et al.** *Agric-Biol. Chem.,* 1990, vol. 54 (7), 1811-1818
- **Milo Gibal.** Biopharmaceutics and Pharmacology. Lea and Sediger, 1991
- **Vinter, J.G.** *J. Comput. Aided Des.,* 1996, vol. 10, 417
- **Covitz, K. M. et al.** *Pharm. Res.,* 1996, vol. 13 (11), 1631-34
- **Temple, C.S. et al.** *J. Physiol. (London),* 1996, vol. 494, 795
- **Meredith, D. et al.** *J. Physiol. (London),* 1998, vol. 512, 629
- **Lynch et al.** *J Cardiovasc.Pharmacol.,* 1981, vol. 3, 49-60
- **X. Guan et al.** *Carcinogenesis,* 1996, vol. 17, 1791-1798
- **R. J. Ruch et al.** *Carcinogenesis,* 1994, vol. 15, 301-306
- **B. V. Madhukar et al.** *Cancer Lett.,* 1996, vol. 106, 117-123
- **W. K. Hong et al.** *Science,* 1997, vol. 278, 1073-1077
- **E. Meier et al.** *Drug Development Research,* 1997, vol. 40, 1-16
- Remington's Pharmaceutical Sciences. Mark Publishing Company, 1985, vol. 17
- **K. M. Abdullah et al.** *Endocrine,* 1999, vol. 10, 35-41
- **M. Saitoh et al.** *Carcinogenesis,* 1997, vol. 18, 1319-1328
- **J. A. Goliger et al.** *Mol.Biol.Cell,* 1995, vol. 6, 1491-1501
- **G. J. Christ et al.** *Braz. J Med Biol.Res.,* 2000, vol. 33, 423-429
- **R. Dermietzel.** *Brain Res. Brain Res. Rev.,* 1998, vol. 26, 176-183
- **H. Aldskogius et al.** *Prog. Neurobiol,* 1998, vol. 55, 1-26
- **D. Mackay et al.** *Am J Hum.Genet,* 1999, vol. 64, 1357-1364
- **S. G. Spanakis et al.** *Invest Ophthalmol.Vis.Sci.,* 1998, vol. 39, 1320-1328
- **B. D. Larsen ; A. Holm.** *Int.J Pept.Protein Res.,* 1994, vol. 43, 1-9